# EUROPEAN PATENT SPECIFICATION

(11) **EP 2 637 653 B1**
(45) Date of publication and mention of the grant of the patent: **10.01.2018**
(21) Application number: 10779765.6
(22) Date of filing: 11.11.2010
(51) Int. Cl.: A61K 31/192, A61K 31/343, A61K 31/4418, A61K 31/454, A61K 45/06, A61P 35/00

(54) **COMPOSITIONS COMPRISING ETHACRYNIC ACID AND LENALIDOMIDE AND/OR THALIDOMIDE FOR USE IN TREATING MYELOMA**
ZUSAMMENSETZUNGEN ENTHALTEND ETHACRYNSÄURE UND LENALIDOMID UND/ODER THALIDOMID ZUR BEHANDLUNG VON MYELOM
COMPOSITIONS CONTENANT DE L'ACIDE ÉTHACRYNIQUE ET DU LÉNALIDOMIDE ET/OU DU THALIDOMIDE POUR LE TRAITEMENT DE MYÉLOME.

(43) Date of publication of application: 18.09.2013
(73) Proprietor: Schmidt-Wolf, Ingo, 53105 Bonn (DE)
(72) Inventor: Schmidt-Wolf, Ingo, 53105 Bonn (DE)
(74) Representative: Letzelter, Felix Phillip
(86) International application number: PCT/EP2010/067280
(87) International publication number: WO 2012/062366

(56) References cited:
- WO-A1-2009/020590
- WO-A1-2010/081486
- WO-A2-03/097052
- WO-A2-2010/014595
- WO-A2-2010/077310
- US-A1- 2006 166 947
- US-A1- 2009 060 903
- LU, DESHENG ET AL: "Ethacrynic acid exhibits selective toxicity to chronic lymphocytic leukemia cells by inhibition of the Wnt /.beta.- catenin pathway", PLOS ONE , 4(12), NO PP. GIVEN CODEN: POLNCL; ISSN: 1932-6203 URL: HTTP://WWW.PLOSONE.ORG/ARTICLE/FETCHOBJECT ATTACHMENT.ACTION?URI=INFO% 3ADOI%2F10.1371%2FJOURNAL.PONE.0008294&REP RESENTATION=PDF, 2009, XP8139823,

## Description

The present invention is directed to compositions for use in the treatment of myeloma, as defined in the claims. The present disclosure is directed to compositions for the treatment of cancer, in particular for the treatment of myeloma, in particular for the treatment of multiple myeloma. The present invention is further directed to the use of said compositions for the manufacture of a medicament for the treatment of myeloma, as defined in the claims. The present disclosure is further directed to the use of said compositions for the manufacture of a medicament for the treatment of said diseases and for the use of said compositions in a medicament for treating of said diseases in animals and humans.

The Wnt signalling pathway describes a network of proteins, most well known for their roles in embryogenesis and the development of cancer, but also involved in normal physiological processes in adult animals.

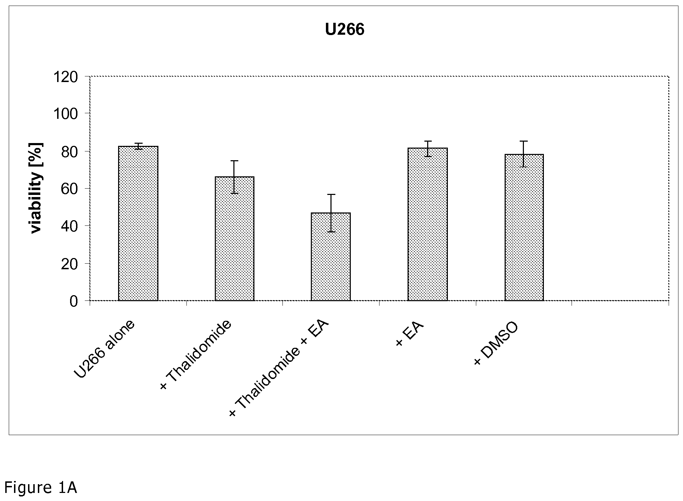

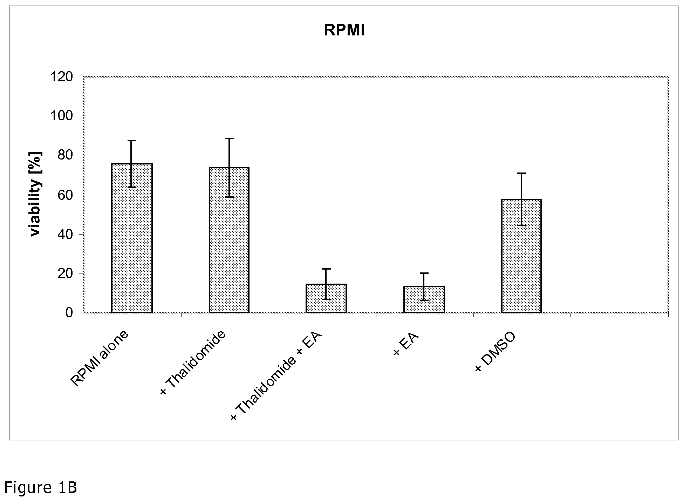

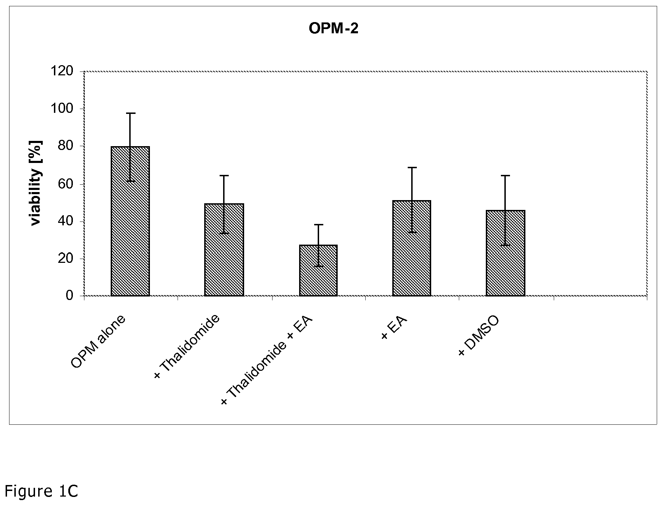

There are 19 known Wingless-Int (Wnt) genes found in the human and mouse genome, which comprise a family of secreted glycoproteins involved in cell proliferation, differentiation, and oncogenesis. The Wnts induce a signaling cascade and regulate early B cell growth and survival by stabilization of β-catenin. Therefore an aberrant activation of the Wnt signaling cascade leads to oncogenesis. In comparison with normal B and T cells, there is an overexpression of the Wnt3 gene detected in B cell chronic lymphocytic leukemia (CLL). The receptor complex of the ligand (Wnt) consists of a receptor of the Frizzled (Fzd) family and of the low-density lipoprotein-receptor-related proteins (LRP) 5 and 6. When Wnt binds to its receptor complex, β-catenin is stabilized by the phosphoprotein Dishevelled (Dvl) which inhibitits the phosphorylating effect of glycogen synthase kinase (GSK)-3β in the Axin complex. The phosphorylated form of β-catenin would be ubiquitinated and degraded by the proteasome. Unphosphorylated β-catenin in the cytoplasm leads to a translocation into the nucleus. Thus, accumulation of unphosphorylated β-catenin in the nucleus activates transcription factors of the T cell (TCF) and lymphoid-enhancing (LEF) family and stimulates the expression of genes such as c-myc, c-jun, fra-1, and cyclin D1. This is the mechanism of the classical or canonical Wnt signaling cascade. The activation of the Wnt/β-catenin pathway can be inhibited by R-etodolac, the R-enantiomer of a nonsteroidal antiinflammatory drug (see Fig. 1). In the β-Catenin pathway, cytoplasmic β-catenin is destabilized by a multi-protein complex containing GSK-3β, CKIa and APC in the absence of Wnt. β-Catenin is phosphorylated by CKIα and GSK-3β efficiently in this complex, and phosphorylated β-catenin is ubiquitinated and degraded by the proteasome. When Wnt binds to its cell surface receptor consisting of Frizzled and LRP5/6, the phosphorylation of β-catenin by GSK-3β is suppressed, and consequently β-catenin is accumulated in the cytosol. The accumulated β-catenin is translocated in the nucleus, where it binds to and activates Tcf/Lef, resulting in expression of the target genes (see Fig. 1).

Aberrant activation of Wnt/β-catenin signaling promotes the development of several cancers. Recently, it has been demonstrated that the Wnt pathway is also activated in myeloma. Therefore, the Wnt/beta catenin signaling molecules are attractive candidates for development of targeted therapies in this disease. To this extent, we recently confirmed that the diuretic agent ethacrynic acid (EA) inhibits Wnt/beta catenin signaling. Patients with myeloma are currently treated with drugs like doxorubicin and thalidomide, or with novel compounds like bortezomib and lenalidomide. Our study was aimed at testing these compounds in combination with EA. Lenalidomide and EA were more effective than thalidomide in decreasing the viability of myeloma cell lines. Interestingly, the addition of thalidomide and lenalidomide indicated a synergistic effect of these drugs with EA. Moreover, we could demonstrate that beta catenin expression is down regulated when EA is added to lymphoma cells. Tumor growth as well as overall survival were significantly reduced in mice treated with EA or EA plus lenalidomide as compared to mice treated with lenalidomide alone. In conclusion, our results reveal a significant selective induction of apoptosis by EA and suggest a synergistic effect when combined with thalidomide and/or lenalidomide in myeloma cells.

Our study was aimed further aimed at testing doxorubicin, thalidomide, bortezomib and lenalidomide in combination with ciclopirox (CIC). Lenalidomide and CIC were more effective than thalidomide in decreasing the viability of myeloma cell lines. Interestingly, the addition of thalidomide and lenalidomide indicated a synergistic effect of these drugs with CIC. Moreover, we could demonstrate that beta catenin expression is down regulated when CIC is added to lymphoma cells. Tumor growth as well as overall survival were significantly reduced in mice treated with CIC or CIC plus lenalidomide as compared to control mice. In conclusion, our results reveal a significant selective induction of apoptosis by CIC and suggest a synergistic effect when combined with thalidomide and/or lenalidomide in myeloma cells.

Multiple myeloma, a well known but still incurable disease, is a hematologic malignancy of B-lymphocytes. While standard chemotherapy regimens have been used for years, novel agents like lenalidomide and bortezomib have become an essential part of today's therapies. Nevertheless, new therapeutical strategies are required in the future. Aberrant activation of Wnt/β-catenin signaling promotes the development of several cancers. Recently, it has been demonstrated that the Wnt pathway is also activated in lymphoma and myeloma. Thus, the Wnt/beta catenin signaling molecules are attractive candidates for development of targeted therapies in these diseases. To this extent, we recently confirmed that the diuretic agent ethacrynic acid (EA) and the antifungal agent ciclopirox olamine (CIC) inhibit Wnt/beta catenin signaling. Piroctone olamine, an ethanolamine salt of the hydroxamic acid derivative piroctone, has very similar chemical features as compared to ciclopirox olamine. Thus, in this study the anti-tumor effect of oral piroctone was investigated in vitro and in vivo in a murine myeloma model.

Griseofulvin has very similar chemical features as compared to ciclopirox olamine. Thus, in this study the anti-tumor effect of oral griseofulvin was investigated in vitro and in vivo in a murine myeloma model.

Thalidomide is a compound of the structural formula

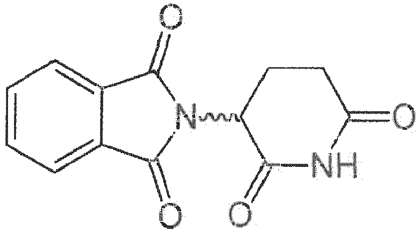

The Systematic (IUPAC) name for thalidomide is (*RS*)-2-(2,6-dioxopiperidin-3-yl)-1*H-*isoindole-1,3(2*H*)-dione.

Thalidomide was introduced as a sedative drug in the late 1950s. In 1961, it was withdrawn due to teratogenicity and neuropathy. There is now a growing clinical interest in thalidomide, and it is introduced as an immunomodulatory agent used primarily, combined with dexamethasone, to treat multiple myeloma.

Lenalidomide (racemate) is a compound of the structural formula

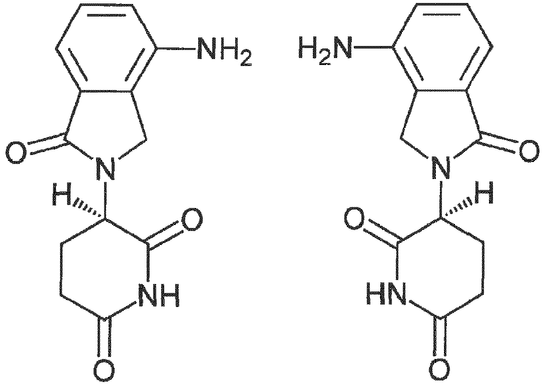

The systematic (IUPAC) name for Lenalidomide is (*RS*)-3-(4-amino-1-oxo-3*H-*isoindol-2-yl)piperidine-2,6-dione.

Lenalidomide, initially known as CC-5013, is a derivative of thalidomide introduced in 2004. It was initially intended as a treatment for multiple myeloma, for which thalidomide is an accepted therapeutic modality, but has also shown efficacy in the class of hematological disorders known as myelodysplastic syndromes (MDS).

Ethacrynic acid is a compound of the structural formula

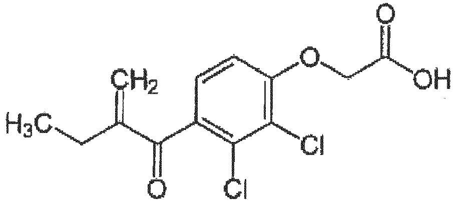

The systematic (IUPAC) name for ethacrynic acid is 2-[2,3-dichloro-4-(2-methylidenebutanoyl) phenoxy] acetic acid.

Ethacrynic acid (EA) is known as a potent diuretic. Therefore, diuresis with water and electrolyte depletion is the consequence, if excessive amounts are administered. EA is an unsaturated ketone derivative of an aryloxyacetic acid and chemically designated as [2,3-dichloro-4-(2-methylene-1-oxobutyl)phenoxy] acetic acid. It is a white, crystalline powder which is slightly solube in water, but soluble in most organic solvents e.g. alcohols, chloroform, and benzene. The sodium salt of EA is Ethacrynate sodium and also available as diuretic agent (Tablets Edecrin (Ethacrynic acid) and intravenous Sodium Edecrin (Ethacrynate sodium) (2005). Merck & Co., Inc. Whitehouse Station, NY, USA). In human tumors as well as in many animal models of carcinogenesis, glutathione-S-transferase P (GST-P) is overexpressed. There are seven classes of glutathione-S-transferases (GSTs) in a family of dimeric enzymes performing multiple functions. One of them is the conjugation of glutathione (GSH) with electrophilic compounds. GSH is a tripeptide of the amino acids glutamine (Glu), cysteine (Cys) and glycine (Gly) forming the compound γ-glutamyl-cysteinyl-glycine. It acts as a reducing agent and as an antioxidant. The conjugation of GSH with toxic compounds forms mercapturates catalyzed by the GSTs. This process forms S-substituted Cys by the release of Glu and Gly residues. Finally, a mercapturic acid which can be excreted in urine is formed by the acetylation of the cysteinyl amino group. The pi-class GSTs, as GST-P in rats and GST P1-1 in humans appear in an association with neoplastic development and anticancer drug resistance. EA is a GST P1-1 inhibitor with the induction of apoptosis in some cell lines. It has been shown to bind GSTs. The in vitro cytotoxicity of chemotherapeutic agents tested in cell lines could be enhanced by EA.

Ciclopirox is a compound of the structural formula

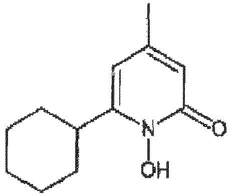

The systematic (IUPAC) name of ciclopirox is 6-cyclohexyl-1-hydroxy-4-methyl-1,2-dihydropyridin-2-one. Ciclopirox is often used as ciclopirox olamine.

Ciclopirox olamine (cic) is a synthetic antifungal agent which is used topical for the treatment of yeast infections in humans (Penlac (ciclopirox) Topical Solution, 8 % (2005), Dermik Laboratories, Berwyn, PA, USA). Ciclopirox is a hydroxypyridone derivative and the free acid of cic. Nevertheless, there is an identical spectrum of activity between cic and ciclopirox (Nail Batrafen, Ciclopirox 8 % Nail Lacquer (1999), Hoechst Marion Roussel (NZ) Limited, Penrose, Auckland). Ciclopirox is chemically designed as 6-Cyclohexyl-1-hydroxy-4-methyl-2(1H)-pyridone. It is a white powder, soluble in methanol. The main metabolic pathway how cic is degraded is glucuronidation based on the secretion of glucuronides (Penlac (ciclopirox) Topical Solution, 8 % (2005), Dermik Laboratories, Berwyn, PA, USA). Cic, as a chelator of polyvalent metal cations (e.g. Fe3+ and AI3+), inhibits the metal-dependent enzymes occurring in the metabolism of a cell (Penlac (ciclopirox) Topical Solution, 8 % (2005), Dermik Laboratories, Berwyn, PA, USA; Nail Batrafen, 1999). It blocks reversibly the cell cycle near the G1/S phase boundary, tested on HL-60 promyeloid leukemia cells.

Piroctone is a compound of the structural formula

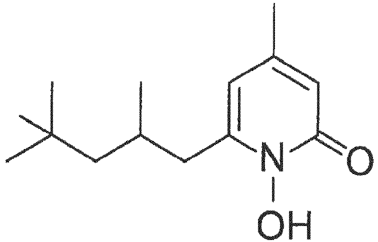

Piroctone is often used in form of piroctone olamine, also known as Octopirox, of the structural formula

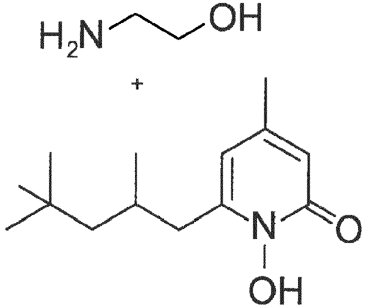

The CAS Number of piroctone olamine is 68890-66-4.

Piroctone olamine, also known as Octopirox and piroctone ethanolamine, is a compound sometimes used in the treatment of fungal infections. Piroctone olamine is the ethanolamine salt of the hydroxamic acid derivative piroctone. It is often used in anti-dandruff shampoo.

Griseofulvin is a compound of the structural formula

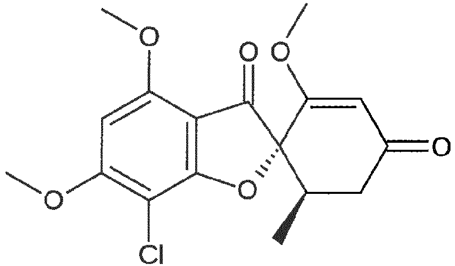

The systematic (IUPAC) name for griseofulvin is (2S,6'R)-7-chloro-2',4,6-trimethoxy-6'-methyl-3H,4'H-spiro[1-benzofuran-2,1'-cyclohex[2]ene]-3,4'-dione.

Griseofulvin (also known as Grisovin) is an antifungal drug that is administered orally. It is used both in animals and in humans, to treat fungal infections of the skin (commonly known as ringworm) and nails. It is derived from the mold Penicillium griseofulvum.

The WO 2010/081486 A1 discloses compositions comprising ethacrynic acid and/or griseofulvin as well as ciclopirox or ciclopirox olamine for the treatment of myeloma. The WO 2010/077310 A2 discloses ethacrynic acid amides for use in the treatment of cancer.

Lu, Desheng et al, PLOS ONE, 4 (12), 2009 discloses that EA is cytotoxic to CLL cells. US-A-2009/060903 and US-A- 2006/166947 disclose *inter alia* the treatment of multiple myeloma with thalidomide. WO-A-03/097052 discloses the use of lenalidomide for the treatment of multiple myeloma, including relapsing multiple myeloma. It was the object underlying the present invention to provide compositions for the treatment of myeloma. A further object of the present invention was to provide the use of such compounds for the manufacture of a medicament for the treatment of myeloma. The object underlying the present invention is solved by a composition, comprising one or more compounds (I) selected from the group consisting of lenalidomide, and thalidomide, and compound (II) ethacrynic acid, for use in the treatment of myeloma.

In a preferred embodiment, the composition described above is adapted for oral, parenteral, rectal, nasal, vaginal, and/or topical administration.

In a preferred embodiment of the composition described above, compound (I) comprises lenalidomide and compound (II) comprises ethacrynic acid.
In a preferred embodiment of the composition described above, compound (I) comprises thalidomide and compound (II) comprises ethacrynic acid.
In a preferred embodiment of the composition described above, compound (I) comprises lenalidomide and thalidomide and compound (II) comprises ethacrynic acid. The object underlying the present invention is also solved by the use of a composition comprising one or more compounds (I) selected from the group consisting of lenalidomide, and thalidomide, and compound (II) ethacrynic acid for the manufacture of a medicament for use in the treatment of myeloma.
In a preferred embodiment of the use described above, compound (I) comprises lenalidomide and compound (II) comprises ethacrynic acid.
In a preferred embodiment of the use described above, compound (I) comprises thalidomide and compound (II) comprises ethacrynic acid.

In a preferred embodiment of the use described above, compounds (I) comprise lenalidomide and thalidomide and compound (II) comprises ethacrynic acid.
In a preferred embodiment of the use described above, the compounds are present in dosage unit form in the medicament.
In a preferred embodiment of the use described above, the dosage unit form comprises approximately 1 to 1000 mg, in particular 10 to 250 mg of lenalidomide, and approximately 1 to 1000 mg, in particular 10 to 250 mg of ethacrynic acid. In a preferred embodiment of the use described above, the dosage unit form comprises approximately 1 to 1000 mg, in particular 10 to 250 mg of thalidomide, and approximately 1 to 1000 mg, in particular 10 to 250 mg of ethacrynic acid. In a preferred embodiment of the use described above, the dosage unit form comprises approximately 1 to 1000 mg, in particular 10 to 250 mg of thalidomide, and approximately 1 to 1000 mg, in particular 10 to 250 mg of lenalidomide, and approximately 1 to 1000 mg, in particular 10 to 250 mg of ethacrynic acid.
Further disclosed is a method for treating cancer, in particular for the treatment of myeloma, lymphoma and leukaemia, in animals and humans, characterized by administering an amount sufficient to treat the cancer of a composition comprising one or more compounds (I) selected from the group consisting of lenalidomide, and thalidomide, and/or pharmaceutically acceptable derivatives thereof and compound (II) ethacrynic acid
and/or pharmaceutically acceptable derivatives thereof.
In a preferred embodiment of the method described above, the composition is adapted for oral, parenteral, rectal, nasal, vaginal, or topical administration.
In a preferred embodiment of the method described above, compound (I) comprises lenalidomide and compound (II) comprises ethacrynic acid.

In a preferred embodiment of the method described above, compound (I) comprises thalidomide and compound (II) comprises ethacrynic acid.
In a preferred embodiment of the method described above, compounds (I) comprise lenalidomide and thalidomide and compound (II) comprises ethacrynic acid.
In a preferred embodiment of the method described above, lenalidomide and/or pharmaceutically acceptable derivatives thereof, is administered at a dose of about 1 to 1000 mg, in particular 10 to 250 mg per day and ethacrynic acid, and/or pharmaceutically acceptable derivatives thereof, is administered at a dose of about 1 to 1000 mg, in particular 10 to 250 mg per day.In a preferred embodiment of the method described above, thalidomide and/or pharmaceutically acceptable derivatives thereof, is administered at a dose of about 1 to 1000 mg, in particular 10 to 250 mg per day and ethacrynic acid, and/or pharmaceutically acceptable derivatives thereof, is administered at a dose of about 1 to 1000 mg, in particular 10 to 250 mg per day.
In a preferred embodiment of the method described above, lenalidomide and/or pharmaceutically acceptable derivatives thereof, is administered at a dose of about 1 to 1000 mg, in particular 10 to 250 mg per day and thalidomide and/or pharmaceutically acceptable derivatives thereof, is administered at a dose of about 1 to 1000 mg, in particular 10 to 250 mg per day
and ethacrynic acid, and/or pharmaceutically acceptable derivatives thereof, is administered at a dose of about 1 to 1000 mg, in particular 10 to 250 mg per day. Further disclosed is a composition comprising one or more compounds (I) selected from the group consisting of lenalidomide, and thalidomide, and/or pharmaceutically acceptable derivatives thereof and compound (II) ciclopirox, and/or pharmaceutically acceptable derivatives thereof for the treatment of cancer, in particular for the treatment of myeloma, lymphoma and leukaemia.
In a preferred embodiment, the composition described above is adapted for oral, parenteral, rectal, nasal, vaginal, and/or topical administration.

In a preferred embodiment, the composition described above compound (I) comprises lenalidomide and compound (II) comprises ciclopirox.
In a preferred embodiment, the composition described above compound (I) comprises thalidomide and compound (II) comprises ciclopirox.
In a preferred embodiment, the composition described above compound (I) comprises lenalidomide and thalidomide and compound (II) comprises ciclopirox. Further disclosed is the use of a composition comprising one or more compounds (I) selected from the group consisting of lenalidomide, and thalidomide, and/or pharmaceutically acceptable derivatives thereof and compound (II) ciclopirox and/or pharmaceutically acceptable derivatives thereof for the manufacture of a medicament for the treatment of cancer, in particular for the treatment of myeloma, lymphoma and leukaemia.

In a preferred embodiment of the use described above, compound (I) comprises lenalidomide and compound (II) comprises ciclopirox.
In a preferred embodiment of the use described above, compound (I) comprises thalidomide and compound (II) comprises ciclopirox.
In a preferred embodiment of the use described above, compounds (I) comprise lenalidomide and thalidomide and compound (II) comprises ciclopirox.
In a preferred embodiment of the use described above, compounds are present in dosage unit form in the medicament.
In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of ciclopirox, and/or pharmaceutically acceptable derivatives thereof.
In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of ciclopirox, and/or pharmaceutically acceptable derivatives thereof.
In a preferred embodiment of the use described above, the dosage unit form comprises approximately 1 to 1000 mg, in particular 10 to 250 mg of thalidomide and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of ciclopirox, and/or pharmaceutically acceptable derivatives thereof.

Further disclosed is a method for treating cancer, in particular for the treatment of myeloma, lymphoma and leukaemia, in animals and humans, characterized by administering an amount sufficient to treat the cancer of a composition comprising one or more compounds (I) selected from the group consisting of lenalidomide, and thalidomide, and/or pharmaceutically acceptable derivatives thereof and compound (II) ciclopirox and/or pharmaceutically acceptable derivatives thereof.

In a preferred embodiment of the method described above, the composition is adapted for oral, parenteral, rectal, nasal, vaginal, or topical administration.

In a preferred embodiment of the method described above, compound (I) comprises lenalidomide and compound (II) comprises ciclopirox.

In a preferred embodiment of the method described above, compound (I) comprises thalidomide and compound (II) comprises ciclopirox.

In a preferred embodiment of the method described above, compounds (I) comprise lenalidomide and thalidomide and compound (II) comprises ciclopirox.

In a preferred embodiment of the method described above, lenalidomide and/or pharmaceutically acceptable derivatives thereof, is administered at a dose of about 1 to 1000 mg, in particular 10 to 250 mg per day and ciclopirox, and/or pharmaceutically acceptable derivatives thereof, is administered at a dose of about 1 to 1000 mg, in particular 10 to 250 mg per day.

In a preferred embodiment of the method described above, thalidomide and/or pharmaceutically acceptable derivatives thereof, is administered at a dose of about 1 to 1000 mg, in particular 10 to 250 mg per day and ciclopirox, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and thalidomide and/or pharmaceutically acceptable derivatives thereof, is administered at a dose of about 1 to 1000 mg, in particular 10 to 250 mg per day
and ciclopirox, and/or pharmaceutically acceptable derivatives thereof, is administered at a dose of about 1 to 1000 mg, in particular 10 to 250 mg per day.

Further disclosed is a composition, comprising one or more compounds (I) selected from the group consisting of lenalidomide, and thalidomide, and/or pharmaceutically acceptable derivatives thereof and compound (II) piroctone, and/or pharmaceutically acceptable derivatives thereof for the treatment of cancer, in particular for the treatment of myeloma, lymphoma and leukaemia.
In a preferred embodiment, the composition described above is adapted for oral, parenteral, rectal, nasal, vaginal, and/or topical administration.
In a preferred dembodiment of the composition described above, compound (I) comprises lenalidomide and compound (II) comprises piroctone.
In a preferred dembodiment of the composition described above, compound (I) comprises thalidomide and compound (II) comprises piroctone.

In a preferred dembodiment of the composition described above, compound (I) comprises lenalidomide and thalidomide and compound (II) comprises piroctone.

Further disclosed is the use of a composition comprising one or more compounds (I) selected from the group consisting of:
lenalidomide, and thalidomide, and/or pharmaceutically acceptable derivatives thereof and compound (II) piroctone and/or pharmaceutically acceptable derivatives thereof for the manufacture of a medicament for the treatment of cancer, in particular for the treatment of myeloma, lymphoma and leukaemia.

In a preferred embodiment of the use described above, compound (I) comprises lenalidomide and compound (II) comprises piroctone.

In a preferred embodiment of the use described above, compound (I) comprises thalidomide and compound (II) comprises piroctone.

In a preferred embodiment of the use described above, compounds (I) comprise lenalidomide and thalidomide and compound (II) comprises piroctone.

In a preferred embodiment of the use described above, compounds are present in dosage unit form in the medicament.

In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of piroctone, and/or pharmaceutically acceptable derivatives thereof.
In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of piroctone, and/or pharmaceutically acceptable derivatives thereof.
In a preferred embodiment of the use described above, the dosage unit form comprises approximately 1 to 1000 mg, in particular 10 to 250 mg of thalidomide and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of piroctone, and/or pharmaceutically acceptable derivatives thereof.

Further disclosed is a method for treating cancer, in particular for the treatment of myeloma, lymphoma and leukaemia, in animals and humans, characterized by administering an amount sufficient to treat the cancer of a composition comprising one or more compounds (I) selected from the group consisting of lenalidomide, and thalidomide, and/or pharmaceutically acceptable derivatives thereof and compound (II) piroctone and/or pharmaceutically acceptable derivatives thereof.
In a preferred embodiment of the method described above, the composition is adapted for oral, parenteral, rectal, nasal, vaginal, or topical administration.
In a preferred embodiment of the method described above, compound (I) comprises lenalidomide and compound (II) comprises piroctone.
In a preferred embodiment of the method described above, compound (I) comprises thalidomide and compound (II) comprises piroctone.

In a preferred embodiment of the method described above, compounds (I) comprise lenalidomide and thalidomide and compound (II) comprises piroctone.
In a preferred embodiment of the method described above, lenalidomide and/or pharmaceutically acceptable derivatives thereof, is administered at a dose of about 1 to 1000 mg, in particular 10 to 250 mg per day and piroctone, and/or pharmaceutically acceptable derivatives thereof, is administered at a dose of about 1 to 1000 mg, in particular 10 to 250 mg per day.
In a preferred embodiment of the method described above, thalidomide and/or pharmaceutically acceptable derivatives thereof, is administered at a dose of about 1 to 1000 mg, in particular 10 to 250 mg per day and piroctone, and/or a pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and thalidomide and/or pharmaceutically acceptable derivatives thereof, is administered at a dose of about 1 to 1000 mg, in particular 10 to 250 mg per day and piroctone, and/or pharmaceutically acceptable derivatives thereof, is administered at a dose of about 1 to 1000 mg, in particular 10 to 250 mg per day.

Further disclosed is a composition, comprising one or more compounds (I) selected from the group consisting of lenalidomide, and thalidomide, and/or pharmaceutically acceptable derivatives thereof and compound (II) griseofulvin, and/or pharmaceutically acceptable derivatives thereof for the treatment of cancer, in particular for the treatment of myeloma, lymphoma and leukaemia.
In a preferred embodiment, the composition described above is adapted for oral, parenteral, rectal, nasal, vaginal, and/or topical administration.
In a preferred embodiment of the composition described above, compound (I) comprises lenalidomide and compound (II) comprises griseofulvin.

In a preferred embodiment of the composition described above, compound (I) comprises thalidomide and compound (II) comprises griseofulvin.

In a preferred embodiment of the composition described above, compound (I) comprises lenalidomide and thalidomide and compound (II) comprises griseofulvin.

Further disclosed is the use of a composition comprising one or more compounds (I) selected from the group consisting of lenalidomide, and thalidomide, and/or pharmaceutically acceptable derivatives thereof and compound (II) griseofulvin and/or pharmaceutically acceptable derivatives thereof for the manufacture of a medicament for the treatment of cancer, in particular for the treatment of myeloma, lymphoma and leukaemia.

In a preferred embodiment of the use described above, compound (I) comprises lenalidomide and compound (II) comprises griseofulvin.
In a preferred embodiment of the use described above, compound (I) comprises thalidomide and compound (II) comprises griseofulvin.
In a preferred embodiment of the use described above, compounds (I) comprise lenalidomide and thalidomide and compound (II) comprises griseofulvin.
In a preferred embodiment of the use described above, the compounds are present in dosage unit form in the medicament.
In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of griseofulvin, and/or pharmaceutically acceptable derivatives thereof.
In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of griseofulvin, and/or pharmaceutically acceptable derivatives thereof.
In a preferred embodiment of the use described above, the dosage unit form comprises approximately 1 to 1000 mg, in particular 10 to 250 mg of thalidomide and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of griseofulvin, and/or pharmaceutically acceptable derivatives thereof.

Further disclosed is a method for treating cancer, in particular for the treatment of myeloma, lymphoma and leukaemia, in animals and humans, characterized by administering an amount sufficient to treat the cancer of a composition comprising one or more compounds (I) selected from the group consisting of lenalidomide, and thalidomide, and/or pharmaceutically acceptable derivatives thereof and compound (II) griseofulvin and/or pharmaceutically acceptable derivatives thereof.
In a preferred embodiment of the method described above, the composition is adapted for oral, parenteral, rectal, nasal, vaginal, or topical administration.
In a preferred embodiment of the method described above, compound (I) comprises lenalidomide and compound (II) comprises griseofulvin.
In a preferred embodiment of the method described above, compound (I) comprises thalidomide and compound (II) comprises griseofulvin.

In a preferred embodiment of the method described above, compounds (I) comprise lenalidomide and thalidomide and compound (II) comprises griseofulvin.
In a preferred embodiment of the method described above, lenalidomide and/or pharmaceutically acceptable derivatives thereof, is administered at a dose of about 1 to 1000 mg, in particular 10 to 250 mg per day and griseofulvin, and/or pharmaceutically acceptable derivatives thereof, is administered at a dose of about 1 to 1000 mg, in particular 10 to 250 mg per day.
In a preferred embodiment of the method described above, thalidomide and/or pharmaceutically acceptable derivatives thereof, is administered at a dose of about 1 to 1000 mg, in particular 10 to 250 mg per day and griseofulvin, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and thalidomide and/or pharmaceutically acceptable derivatives thereof, is administered at a dose of about 1 to 1000 mg, in particular 10 to 250 mg per day and griseofulvin, and/or pharmaceutically acceptable derivatives thereof, is administered at a dose of about 1 to 1000 mg, in particular 10 to 250 mg per day.

Further disclosed is a composition, comprising one or more compounds (I) selected from the group consisting of lenalidomide, and thalidomide, and/or pharmaceutically acceptable derivatives thereof and one or more compounds (II) selected from the group consisting of ethacrynic acid, ciclopirox, piroctone, and griseofulvin, and/or pharmaceutically acceptable derivatives thereof for the treatment of cancer, in particular for the treatment of myeloma, lymphoma and leukaemia.
In a preferred embodiment, the composition described above is adapted for oral, parenteral, rectal, nasal, vaginal, and/or topical administration.
In a preferred embodiment of the composition described above, compound (I) comprises lenalidomide and compound (II) comprises ciclopirox.
In a preferred embodiment of the composition described above, compound (I) comprises thalidomide and compound (II) comprises ciclopirox.
In a preferred embodiment of the composition described above, compound (I) comprises lenalidomide and compound (II) comprises ethacrynic acid.

In a preferred embodiment of the composition described above, compound (I) comprises thalidomide and compound (II) comprises ethacrynic acid.
In a preferred embodiment of the composition described above, compound (I) comprises lenalidomide and thalidomide and compound (II) comprises ethacrynic acid. In a preferred embodiment of the composition described above, compound (I) comprises lenalidomide and thalidomide and compound (II) comprises ciclopirox.
In a preferred embodiment of the composition described above, compound (I) comprises lenalidomide and compound (II) comprises griseofulvin.

In a preferred embodiment of the composition described above, compound (I) comprises thalidomide and compound (II) comprises griseofulvin.
In a preferred embodiment of the composition described above, compound (I) comprises lenalidomide and compound (II) comprises piroctone.
In a preferred embodiment of the composition described above, compound (I) comprises thalidomide and compound (II) comprises piroctone.
In a preferred embodiment of the composition described above, compounds (I) comprise lenalidomide and thalidomide and compound (II) comprises piroctone.
In a preferred embodiment of the composition described above, compounds (I) comprise lenalidomide and thalidomide and compound (II) comprises griseofulvin.

In a preferred embodiment of the composition described above, ciclopirox is in form or ciclopirox olamine.
In a preferred embodiment of the composition described above, piroctone is in form of piroctone olamine.

Further disclosed is the use of a composition comprising one or more compounds (I) selected from the group consisting of lenalidomide, and thalidomide, and/or pharmaceutically acceptable derivatives thereof and one or more compounds (II) selected from the group consisting of ethacrynic acid, ciclopirox, piroctone, and griseofulvin, and/or pharmaceutically acceptable derivatives thereof for the manufacture of a medicament for the treatment of cancer, in particular for the treatment of myeloma, lymphoma and leukaemia.
In a preferred embodiment of the use described above, compound (I) comprises lenalidomide and compound (II) comprises ciclopirox.
In a preferred embodiment of the use described above, compound (I) comprises thalidomide and compound (II) comprises ciclopirox

In a preferred embodiment of the use described above, compound (I) comprises lenalidomide and compound (II) comprises ethacrynic acid.

In a preferred embodiment of the use described above, compound (I) comprises thalidomide and compound (II) comprises ethacrynic acid.

In a preferred embodiment of the use described above, compounds (I) comprise lenalidomide and thalidomide and compound (II) comprises ethacrynic acid.

In a preferred embodiment of the use described above, compounds (I) comprise lenalidomide and thalidomide and compound (II) comprises ciclopirox.

In a preferred embodiment of the use described above, compound (I) comprises lenalidomide and compound (II) comprises griseofulvin.

In a preferred embodiment of the use described above, compound (I) comprises thalidomide and compound (II) comprises griseofulvin.

In a preferred embodiment of the use described above, compound (I) comprises lenalidomide and compound (II) comprises piroctone.

In a preferred embodiment of the use described above, compound (I) comprises thalidomide and compound (II) comprises piroctone.

In a preferred embodiment of the use described above, compounds (I) comprise lenalidomide and thalidomide and compound (II) comprises piroctone.

In a preferred embodiment of the use described above, compounds (I) comprise lenalidomide and thalidomide and compound (II) comprises griseofulvin.

In a preferred embodiment of the use described above, the compounds are present in dosage unit form in the medicament.

In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of ethacrynic acid, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of ethacrynic acid, and/or pharmaceutically acceptable derivatives thereof.

In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of ciclopirox olamine, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of ciclopirox olamine, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of ciclopirox olamine, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of ethacrynic acid, and/or pharmaceutically acceptable derivatives thereof.

In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of piroctone, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of piroctone, and/or pharmaceutically acceptable derivatives thereof.

In a preferred embodiment of the use described above, the dosage unit form comprises approximately 1 to 1000 mg, in particular 10 to 250 mg of lenalidomide, and/or pharmaceutically acceptable derivatives thereof, and approximately 1 to 1000 mg, in particular 10 to 250 mg of griseofulvin, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of griseofulvin, and/or a pharmaceutically acceptable derivative thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of griseofulvin, and/or pharmaceutically acceptable derivatives thereof.

In a preferred embodiment of the use described above, the dosage unit form comprises approximately 1 to 1000 mg, in particular 10 to 250 mg of thalidomide, and/or pharmaceutically acceptable derivatives thereof, and approximately 1 to 1000 mg, in particular 10 to 250 mg of lenalidomide, and/or pharmaceutically acceptable derivatives thereof, and approximately 1 to 1000 mg, in particular 10 to 250 mg of piroctone, and/or pharmaceutically acceptable derivatives thereof.

Further disclosed is a method for treating cancer, in particular for the treatment of myeloma, lymphoma and leukaemia, in animals and humans, characterized by administering an amount sufficient to treat the cancer of a composition comprising one or more compounds (I) selected from the group consisting of lenalidomide, and thalidomide,
and/or pharmaceutically acceptable derivatives thereof and one or more compounds (II) selected from the group consisting of ethacrynic acid, ciclopirox, piroctone, and griseofulvin, and/or pharmaceutically acceptable derivatives thereof.

In a preferred embodiment of the method described above, the composition is adapted for oral, parenteral, rectal, nasal, vaginal, or topical administration.

In a preferred embodiment of the method described above, compound (I) comprises lenalidomide and compound (II) comprises ciclopirox.

In a preferred embodiment of the method described above, compound (I) comprises thalidomide and compound (II) comprises ciclopirox.

In a preferred embodiment of the method described above, compound (I) comprises lenalidomide and compound (II) comprises ethacrynic acid.

In a preferred embodiment of the method described above, compound (I) comprises thalidomide and compound (II) comprises ethacrynic acid.

In a preferred embodiment of the method described above, compounds (I) comprise lenalidomide and thalidomide and compound (II) comprises ethacrynic acid.

In a preferred embodiment of the method described above, compounds (I) comprise lenalidomide and thalidomide and compound (II) comprises ciclopirox.

In a preferred embodiment of the method described above, compound (I) comprises lenalidomide and compound (II) comprises griseofulvin.

In a preferred embodiment of the method described above, compound (I) comprises thalidomide and compound (II) comprises griseofulvin.

In a preferred embodiment of the method described above, compound (I) comprises lenalidomide and compound (II) comprises piroctone.

In a preferred embodiment of the method described above, compound (I) comprises thalidomide and compound (II) comprises piroctone.

In a preferred embodiment of the method described above, compounds (I) comprise lenalidomide and thalidomide and compound (II) comprises piroctone.

In a preferred embodiment of the method described above, compounds (I) comprise lenalidomide and thalidomide and compound (II) comprises griseofulvin.

In a preferred embodiment of the method described above, lenalidomide an/or pharmaceutically acceptable derivatives thereof, is administered at a dose of about 1 to 1000 mg, in particular 10 to 250 mg per day and ciclopirox, and/or pharmaceutically acceptable derivatives thereof, is administered at a dose of about 1 to 1000 mg, in particular 10 to 250 mg per day.

In a preferred embodiment of the method described above, thalidomide and/or pharmaceutically acceptable derivatives thereof, is administered at a dose of about 1 to 1000 mg, in particular 10 to 250 mg per day and ciclopirox, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and ethacrynic acid, and/or pharmaceutically acceptable derivatives thereof, is administered at a dose of about 1 to 1000 mg, in particular 10 to 250 mg per day.

In a preferred embodiment of the method described above, thalidomide and/or pharmaceutically acceptable derivatives thereof, is administered at a dose of about 1 to 1000 mg, in particular 10 to 250 mg per day and ethacrynic acid, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and thalidomide, and/or pharmaceutically acceptable derivatives thereof, is administered at a dose of about 1 to 1000 mg, in particular 10 to 250 mg per day and ethacrynic acid, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and thalidomide, and/or pharmaceutically acceptable derivatives thereof, is administered at a dose of about 1 to 1000 mg, in particular 10 to 250 mg per day
and ciclopirox, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and griseofulvin, and/or pharmaceutically acceptable derivatives thereof, is administered at a dose of about 1 to 1000 mg, in particular 10 to 250 mg per day.

In a preferred embodiment of the method described above, thalidomide and/or pharmaceutically acceptable derivatives thereof, is administered at a dose of about 1 to 1000 mg, in particular 10 to 250 mg per day and griseofulvin, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and piroctone, and/or pharmaceutically acceptable derivatives thereof, is administered at a dose of about 1 to 1000 mg, in particular 10 to 250 mg per day.

In a preferred embodiment of the method described above, thalidomide and/or pharmaceutically acceptable derivatives thereof, is administered at a dose of about 1 to 1000 mg, in particular 10 to 250 mg per day and piroctone, and/or pharmaceutically acceptable derivatives thereof, is administered at a dose of about 1 to 1000 mg, in particular 10 to 250 mg per day.

In a preferred embodiment of the method described above, lenalidomide and/or pharmaceutically acceptable derivatives thereof, is administered at a dose of about 1 to 1000 mg, in particular 10 to 250 mg per day and thalidomide and/or pharmaceutically acceptable derivatives thereof, is administered at a dose of about 1 to 1000 mg, in particular 10 to 250 mg per day
and piroctone, and/or pharmaceutically acceptable derivatives thereof, is administered at a dose of about 1 to 1000 mg, in particular 10 to 250 mg per day.
In a preferred embodiment of the method described above, lenalidomide and/or pharmaceutically acceptable derivatives thereof, is administered at a dose of about 1 to 1000 mg, in particular 10 to 250 mg per day and thalidomide and/or pharmaceutically acceptable derivatives thereof, is administered at a dose of about 1 to 1000 mg, in particular 10 to 250 mg per day and griseofulvin, and/or pharmaceutically acceptable derivatives thereof, is administered at a dose of about 1 to 1000 mg, in particular 10 to 250 mg per day.

Further disclosed is a composition, comprising piroctone and/or pharmaceutically acceptable derivatives thereof for the treatment of cancer, in particular for the treatment of myeloma, lymphoma and leukaemia.

In a preferred embodiment, the composition described above is adapted for oral, parenteral, rectal, nasal, vaginal, and/or topical administration.
In a preferred embodiment of the composition described above, piroctone is in form of piroctone olamine.
Further disclosed is the use of a composition comprising piroctone and/or pharmaceutically acceptable derivatives thereof for the manufacture of a medicament for the treatment of cancer, in particular for the treatment of myeloma, lymphoma and leukaemia.
In a preferred embodiment of the use described above, the compounds are present in dosage unit form in the medicament.
In a preferred embodiment of the use described above, the dosage unit form comprises approximately 1 to 1000 mg, in particular 10 to 250 mg of prioctone. Further disclosed is a method for treating cancer, in particular for the treatment of myeloma, lymphoma and leukaemia, in animals and humans, characterized by administering an amount sufficient to treat the cancer of a composition comprising piroctone, and/or pharmaceutically acceptable derivatives thereof.

In a preferred embodiment of the method described above, the composition is adapted for oral, parenteral, rectal, nasal, vaginal or tropical administration.

In a preferred embodiment of the method described above, piroctone and/or a pharmaceutically acceptable derivative thereof is administered at a dose of about 1 to 1000 mg, in particular 10 to 250 mg of prioctone per day.

Further disclosed is a composition, comprising griseofulvin and/or pharmaceutically acceptable derivatives thereof for the treatment of cancer, in particular for the treatment of myeloma, lymphoma and leukaemia.

In a preferred embodiment, the composition described above is adapted for oral, parenteral, rectal, nasal, vaginal, and/or topical administration.

Further disclosed is the use of a composition comprising griseofulvin and/or pharmaceutically acceptable derivatives thereof for the manufacture of a medicament for the treatment of cancer, in particular for the treatment of myeloma, lymphoma and leukaemia.

In a preferred embodiment of the use described above, the compounds are present in dosage unit form in the medicament.

In a preferred embodiment of the use described above, the dosage unit form comprises approximately 1 to 1000 mg, in particular 10 to 250 mg of griseofulvin.

Further disclosed is a method for treating cancer, in particular for the treatment of myeloma, lymphoma and leukaemia, in animals and humans, characterized by administering an amount sufficient to treat the cancer of a composition comprising griseofulvin, and/or pharmaceutically acceptable derivatives thereof.

In a preferred embodiment of the method described above, the composition is adapted for oral, parenteral, rectal, nasal, vaginal or tropical administration.

In a preferred embodiment of the method described above, griseofulvin and/or a pharmaceutically acceptable derivative thereof is administered at a dose of about 1 to 1000 mg, in particular 10 to 250 mg of griseofulvin per day.

Further disclosed is a composition, comprising ethacrynic acid and/or pharmaceutically acceptable derivatives thereof for the treatment of cancer, in particular for the treatment of myeloma, lymphoma and leukaemia.

In a preferred embodiment, the composition described above is adapted for oral, parenteral, rectal, nasal, vaginal, and/or topical administration.

Further disclosed is the use of a composition comprising ethacrynic acid and/or pharmaceutically acceptable derivatives thereof for the manufacture of a medicament for the treatment of cancer, in particular for the treatment of myeloma, lymphoma and leukaemia.

In a preferred embodiment of the use described above, the compounds are present in dosage unit form in the medicament.

In a preferred embodiment of the use described above, the dosage unit form comprises approximately 1 to 1000 mg, in particular 10 to 250 mg of ethacrynic acid.

Further disclosed is a method for treating cancer, in particular for the treatment of myeloma, lymphoma and leukaemia, in animals and humans, characterized by administering an amount sufficient to treat the cancer of a composition comprising ethacrynic acid, and/or pharmaceutically acceptable derivatives thereof.

In a preferred embodiment of the method described above, the composition is adapted for oral, parenteral, rectal, nasal, vaginal or tropical administration.

In a preferred embodiment of the method described above, ethacrynic acid and/or a pharmaceutically acceptable derivative thereof is administered at a dose of about 1 to 1000 mg, in particular 10 to 250 mg of ethacrynic acid per day.

Further disclosed is a composition, comprising ciclopirox and/or pharmaceutically acceptable derivatives
thereof for the treatment of cancer, in particular for the treatment of myeloma, lymphoma and leukaemia.
In a preferred embodiment, the composition described above is adapted for oral, parenteral, rectal, nasal, vaginal, and/or topical administration.
In a preferred embodiment of the composition described above, ciclopirox is in form of ciclopirox olamine.
Further disclosed is the use of a composition comprising ciclopirox and/or pharmaceutically acceptable derivatives thereof for the manufacture of a medicament for the treatment of cancer, in particular for the treatment of myeloma, lymphoma and leukaemia.
In a preferred embodiment of the use described above, the compounds are present in dosage unit form in the medicament.
In a preferred embodiment of the use described above, the dosage unit form comprises approximately 1 to 1000 mg, in particular 10 to 250 mg of ciclopirox.

Further disclosed is a method for treating cancer, in particular for the treatment of myeloma, lymphoma and leukaemia, in animals and humans, characterized by administering an amount sufficient to treat the cancer of a composition comprising ciclopirox, and/or pharmaceutically acceptable derivatives thereof.

In a preferred embodiment of the method described above, the composition is adapted for oral, parenteral, rectal, nasal, vaginal or tropical administration.
In a preferred embodiment of the method described above, ciclopirox and/or a pharmaceutically acceptable derivative thereof is administered at a dose of about 1 to 1000 mg, in particular 10 to 250 mg of ciclopirox per day.

The present invention is directed to a composition comprising a) one or more compounds (I) selected from the group consisting of lenalidomide and thalidomide, and b) compound (II) ethacrynic acid for use in the treatment of myeloma, in particular for the treatment of multiple myeloma. The present invention is further directed to the use of said compositions for the manufacture of a medicament for the treatment of myeloma, in particular for the treatment of multiple myeloma. Further disclosed is a method for treating a disease, in particular for treating cancer, in particular for treating myeloma, lymphoma and leukaemia, in particular for treating myeloma, in particular for treating multiple myeloma, characterized by administering an amount sufficient to treat the disease of any of the compositions described above.

### Increased In vivo Efficancy Of Lenalidomide And Thalidomide By Addition Of Ethacrynic acid Targeting The Wnt/Beta-catenin Pathway

Major progress has been achieved in the treatment of multiple myeloma by the introduction of novel agents like thalidomide, lenalidomide and bortezomib. Nevertheless, myeloma remains an incurable disease. In newly diagnosed patients, the combination of lenalidomide and dexamethasone has a response rate of 91%.
Several groups have shown that the Wnt/beta catenin pathway plays an important role in the regulation of cell proliferation, differentiation and apoptosis¹⁻³. Aberrant activation of the Wnt signaling pathway has major oncogenic effects⁴⁻⁷. In the canonical Wnt pathway, the secreted Wnt proteins bind to a receptor complex, consisting of a member of the Frizzled (Fzd) family, and the low-density lipoprotein-receptor-related proteins (LRP) 5 or LRP6. Subsequently the cytoplasmic adaptor protein disheveled (Dvl) is phosphorylated and inhibits glycogen synthase kinase (GSK)-3β activity through its association with axin. Unphosphorylated β-catenin accumulates in the cytoplasm and translocates into the nucleus, where it interacts with T cell (TCF) and lymphoid-enhancing (LEF) factors to activate transcription of Wnt target genes^{4,5,8}. In addition, it has been demonstrated that the Wnt pathway is activated in lymphoma. Therefore, the Wnt/beta catenin signaling molecules are attractive candidates for development of targeted approaches in lymphoma treatment.
In more recent work, our group confirmed that the diuretic agent ethacrynic acid (EA) and the antifungal agent ciclopirox olamine (cic) inhibit Wnt/beta catenin signaling⁹. EA is already used clinically as a diuretic agent. The glutathione-S-transferase (GST) which is over expressed in human tumors in form of GST-P binds glutathione (GSH) to electrophilic compounds leading to detoxification of the cell¹⁰⁻¹³. GSH is a reducing agent and an antioxidant. The binding of EA to GSH can enhance the cytotoxicity of chemotherapeutic agents^{14,15}.

For all of these reasons, we tested the effect of EA on myeloma cells in addition to the commonly used therapeutic drugs. Of the following experimental part, only the experiments relating to the combinations of lenalidomide and/or thalidomide with ethacrynic acid for the treatment of myeloma fit in the scope of the claims. The other experiments are for reference.

### Design and Methods

### Cell lines and culture conditions

The lymphoma cell lines LAM-53, SU-DHL-4, Daudi and Raji, as well as the myeloma cell lines OPM-2, RPMI-8226 and U-266 (all obtained from DSMZ, Collection of Microorganisms and Cell Culture, Braunschweig, Germany) were cultured in RPMI-1640 Medium consisting of 10 % heat-inactivated fetal calf serum (FCS, Invitrogen, Karlsruhe, Germany), 2.5 % 1 M HEPES, and 1x penicillin/streptomycin (all from PAA Laboratories GmbH, Cölbe, Germany). Cells were cultured at a density of 3.3 x 10⁵ cells ml⁻¹ and incubated at 37°C with 5 % CO₂ and 95% humidity.
MPC11 (DMSZ, Braunschweig, Germany) is a murine plasmocytoma cell line derived from the Balb/c strain expressing IgG2b. Cells were cultured in RPMI 1640 medium (PAA Laboratories GmbH, Austria) supplemented with 5% fetal calf serum (FCS), 2 mM glutamine (both from PAA, Cölbe, Germany), 100 U/ml penicillin / 100 U/ml streptomycin (both from Seromed, Jülich, Germany) at 37°C in a humidified 5%CO₂ atmosphere.

### Human samples

Peripheral blood mononuclear cells (PBMC) and PBL were isolated from blood samples of healthy volunteers by Ficoll density gradient centrifugation (Lymphoprep, Nycomed, Oslo, Norway). In addition, bone marrow samples from patients with myeloma were obtained. Ethics approval had been obtained according to the guidelines of our institution.

### Drugs and chemical reagents

In our experiments the following drugs were used: ethacrynic acid (EA; Sigma-Aldrich GmbH, Seelze, Germany), doxorubicin (Cellpharm GmbH, Germany), rituximab (Roche Pharma AG, Grenzach Wyhlen, Germany), bortezomib (Janssen Cilag, Neuss, Germany), thalidomide (Grünenthal Pharma GmbH, Aachen, Germany). Lenalidomide was obtained from Celgene (Munich, Germany). All drugs were tested at various concentrations for 24 - 72 hours.

### DiOC₆ and PI-staining

1x10⁵ Cells were cultured in 3 ml Medium in 6-well plates. EA was dissolved in DMSO, and added in an optimized concentration of 30 µM (EA) alone or in combination with the therapeutic agents at various concentrations for three days. The apoptosis assay was performed with 3'3-dihexyloxacarbocyanine iodide (DiOC₆) detecting mitochondrial membrane potential in viable cells, and propidium iodide (PI) which binds to DNA in necrotic cells, measured by a fluorescence-activated cell sorter (FACS).

For FACS analysis, 500µl staining solution containing 80 nM DiOC₆ in FACS buffer, consisting of deficient RPMI-medium with 0.5% bovine serum albumin (BSA), was mixed with equal volumes of the cell sample in a glass tube and incubated at 37°C for 15 min. After a washing step with PBS/BSA 1% the cells were re-suspended in 500µl PBS/BSA 1%. After addition of 5µl PI-solution (100µg/ml) the cells were analyzed by FACS. Using this assay, viable cells reveal high fluorescence intensity for DiOC₆ and a low expression for PI. In contrary, apoptotic cells show a low expression for DiOC₆ and also a low expression for PI (2). Finally, necrotic cells show a low expression for DiOC₆ and a high expression for PI.

A mean IC₅₀ value in myeloma cells was determined using the mean of the IC₅₀ results determined in OPM-2, U266 and RPMI 8226 cells.

### Isolation of peripheral blood mononuclear cells (PBMCs)

PBMCs were isolated from blood of healthy donors by Ficoll-Hypaque density gradient centrifugation. Blood from buffy coats was mixed 1:2 with PBS/1% BSA (both PAA, Cölbe, Germany) and used for a ficoll gradient (LymphoPrep, PAA, Cölbe, Germany). After the centrifugation at 800 g for 30 minutes, the leukocyte layer was removed and transferred to new tubes. Subsequently, these cells were washed 3 times with PBS/1%BSA and re-suspended in fresh medium, consisting of RPMI medium (PAA, Cölbe, Germany) with 10% fetal calf serum (Invitrogen, Karlsruhe, Germany), 2.5% hepes buffer solution (PAA, Cölbe, Germany), and 1% penicillin/streptomycin (PAA, Cölbe, Germany).

### Bone marrow samples

Bone marrow samples from patients with myeloma were taken after informed consent, single cell suspensions generated and cells ficolled. Cells were incubated with or without EA for three days and measured for viability.

### Western blot

We analyzed the effect of EA on the Wnt/beta catenin pathway by Western blot. Western blot was performed as described recently¹⁶.

### Animal studies

All animal experiments were done at least in duplicate with groups of 6 BALB/c mice (Charles River, Sulzfeld, Germany). 5x10⁵ MCP11 murine myeloma cells were injected per mouse subcutaneously into Balb/c mice. Mice were treated orally with 450µg/day EA and 750 µg lenalidomide/day, respectively. Overall survival and tumor growth were measured. Tumor volume was calculated as follows: volume = length x width² x 0,52. Animals were killed when tumor volume reached 2000 mm³.

### Statistical analysis

For statistical analysis, the numbers of the results comprising the relative viability are expressed as the mean ± standard error of the mean (SEM). Different sample sizes (n) were chosen for different cell lines. Student's t test was used for statistical analysis. A p value below 0.05 was considered significant. Statistical survival analyses were performed with the software GraphPad InStat, Version 3.0.0 (GraphPad Software, San Diego, CA), applied the Mann-Whitney test (non-paired, non-parametric).

### Results

### Inhibition of Wnt/beta catenin signaling by EA

In our recent research we used a 96-well plate-based TOPflash reporter system to screen the Gen-plus drug library (Microsource) that contained 960 compounds. The screen identified EA and cic as Wnt/beta catenin inhibitors¹⁶. Since the canonical Wnt signaling pathway is activated in lymphoma and myeloma cells we further investigated if EA could induce apoptosis and decrease viability of lymphoma and myeloma cell lines, respectively.

### Titration of EA

As a first step, we determined the optimal concentration of EA in various lymphoma and myeloma cell lines. Subsequently, we determined the optimal concentration of EA in PBMCs derived from healthy individuals in order to check for toxicity. Determination of the optimal concentration of EA revealed that 30 µM EA was the most effective concentration to initialize cell death in both lymphoma and myeloma cell lines, without significantly deteriorating viability of normal PBMCs (data not shown). The effect of DMSO as a toxic solvent was only observed in the myeloma cell line OPM-2 (Figure 1C). As a control, peripheral blood mononuclear cells (PBMCs) were investigated by FACS analysis. A toxic effect affecting the cells induced a shift from viable, DiOC₆-positive cells to apoptotic, DiOC₆-negative cells.

### Viability of cell lines and PBMC after 72 hours of culture

Viability decreased slowly over time. After 72 h, the relative viability for the lymphoma cell lines SU-DHL-4, LAM-53 and Raji in the presence of EA (30 µM) was 97.6 +/- 1.1%, 91.0 +/- 4.9% and 78.9 +/- 2.1%, respectively¹⁷. These values were similar to values of the control of PBMC with 89.5 +/- 3.1%.

### Effect of DMSO on viability of cell lines

In contrast to PBMC and lymphoma cell lines, the myeloma cell line OPM-2 was highly sensitive towards DMSO and showed a decrease of relative viability to 66.8 +/- 2.3%.

### Effect of doxorubicin in combination with EA on viability of lymphoma cell lines

In general, lymphoma patients are treated with doxorubicin and rituximab besides other drugs. Therefore, we tested these drugs in lymphoma cells in combination with EA.

In Daudi and Raji cells, the addition of doxorubicin led to a significant decrease in viability of Daudi and Raji cells. However, the combination of EA plus doxorubicin did not further decrease viability of Raji cells. Therefore, we would exclude a synergistic effect (data not shown).

### Effect of rituximab in combination with EA on viability of lymphoma cell lines

In Daudi and Raji cells, the addition of rituximab led to significantly decreased viability of these cells. However, the combination of EA plus rituximab did not further decrease viability of Daudi and Raji cells. Like with doxorubicin, we excluded a synergistic effect of this combination (data not shown).

### Effect of bortezomib in combination with EA on viability of myeloma cell line OPM-2

Treatment of myeloma has changed in recent times. More and more patients are treated with novel drugs, such as bortezomib, lenalidomide and thalidomide - the latter already is known for quite some time. - Therefore, we tested these drugs in OPM-2 myeloma cells in combination with EA.

In OPM-2 cells, the addition of bortezomib led to a significant decrease in viability of OPM-2 myeloma cells. However, the combination of EA plus bortezomib did not further decrease viability of OPM cells. We excluded a synergistic effect of this combination, as well (data not shown).

### Effect of thalidomide, lenalidomide and EA on viability of myeloma cell lines

All three compounds significantly (p<0.05) decreased the viability of myeloma cell lines in vitro (Table I). A mean IC₅₀ value in myeloma cells was determined: Lenalidomide (16.3uM) and EA (30uM) were more effective than thalidomide (>251uM; Table I).

In contrast the effect on normal PBMC and PBL was marginal in EA and lenalidomide. Here, no effect was found for thalidomide (Table I).

### Effect of thalidomide, lenalidomide and EA on viability of lymphoma cell lines

Only EA significantly (p<0.05) decreased the viability of lymphoma cell lines in vitro (Table I). In contrast, lenalidomide and thalidomide showed no effect (Table I).

### Effect of thalidomide in combination with EA on viability of myeloma cell lines

In OPM-2, U-266 and RPMI-8226 cells, the addition of thalidomide led to a significant decrease in viability of OPM-2, U-266 and RPMI-8226 myeloma cells. Interestingly, the combination of EA plus thalidomide led to a further decrease of viability of OPM-2, U-266 and RPMI-8226 cells; suggesting a synergistic effect for this combination (Figure 1).

### Effect of lenalidomide in combination with EA on viability of myeloma cell lines

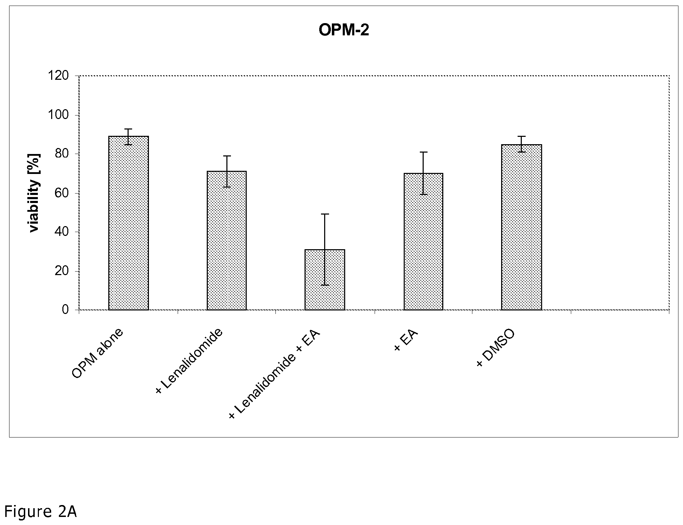

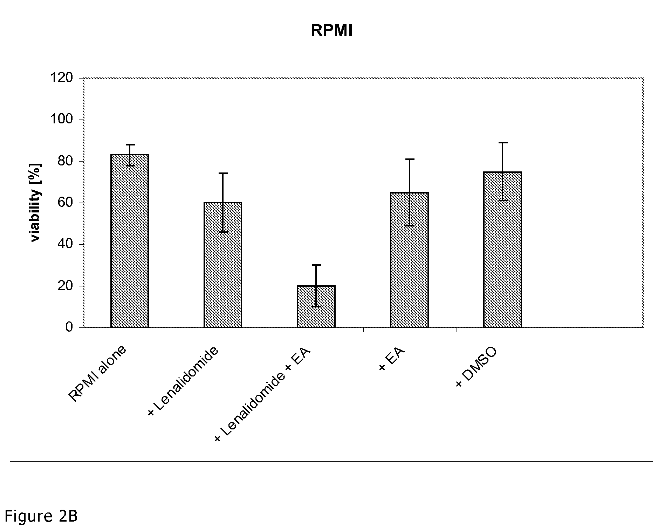

In OPM-2 and RPMI-8226 cells, the addition of lenalidomide led to a significant decrease in viability of OPM-2 and RPMI-8226 myeloma cells. Like with thalidomide, the addition of lenalidomide to EA led to further decreased viability of both OPM-2 and RPMI-8226 cells. These results indicate a synergistic effect for this combination (Figure 2).

### Effect of EA on the Wnt/beta catenin pathway in lymphoma cells

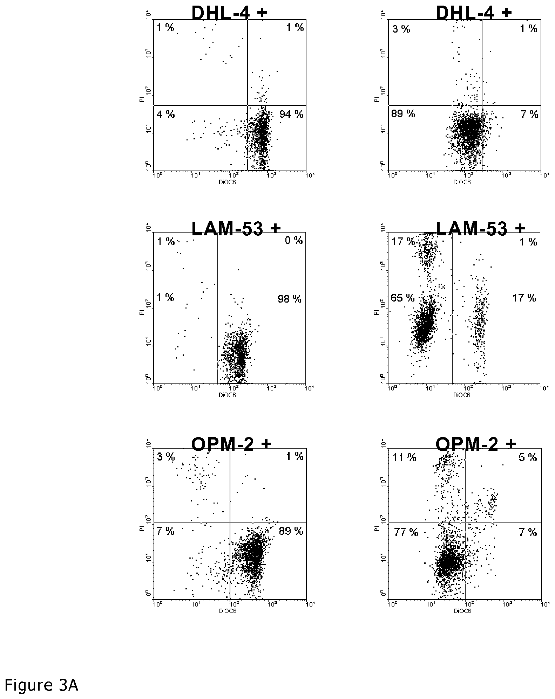

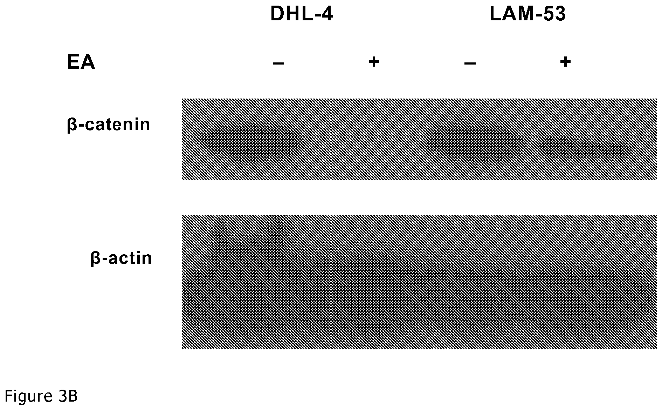

As a next step, we analyzed the effect of EA on the Wnt/beta catenin pathway. The myeloma cell line OPM-2 did not express enough beta catenin compared to lymphoma cell lines. However, we could show that beta catenin is down regulated when EA is added to lymphoma cells (Figure 3).

### Effect of EA on the Wnt/beta catenin pathway in myeloma cells

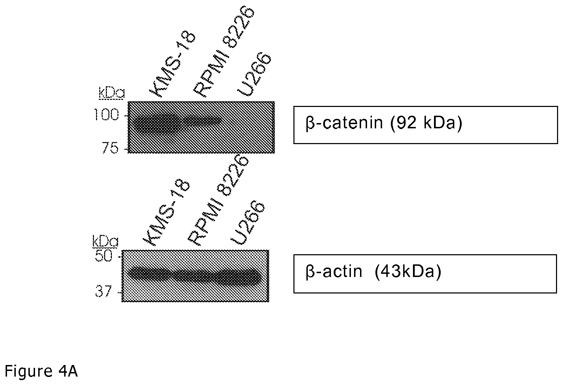

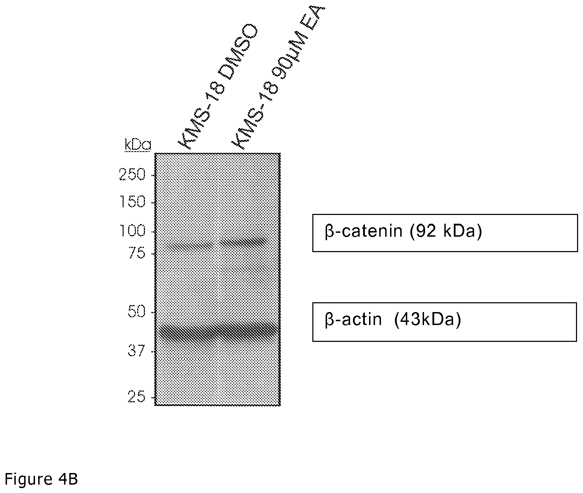

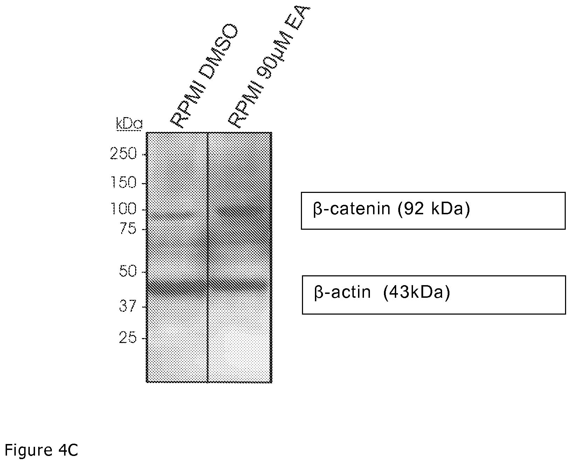

The effect of EA on β-catenin expression in myeloma cells was tested. KMS18 and RPMI cells were found to be β-catenin positive in contrast to U266 cells being negative (Figure 4A). When exposed to EA, β-catenin expression increased in KMS-18 cells (Figure 4B) and slightly increased in RPMI 8226 cells (Figure 4C).

### Effect of EA on viability of myeloma primary cells

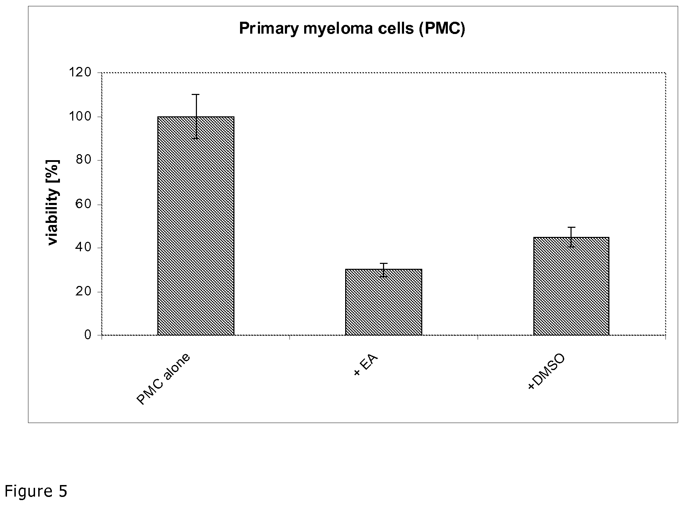

Preliminary data suggest that EA inhibits the growth of primary myeloma cells derived from bone marrow from patients with multiple myeloma (Figure 5).

### Effect of lenalidomide, EA and of the combination of lenalidomide and EA in vivo

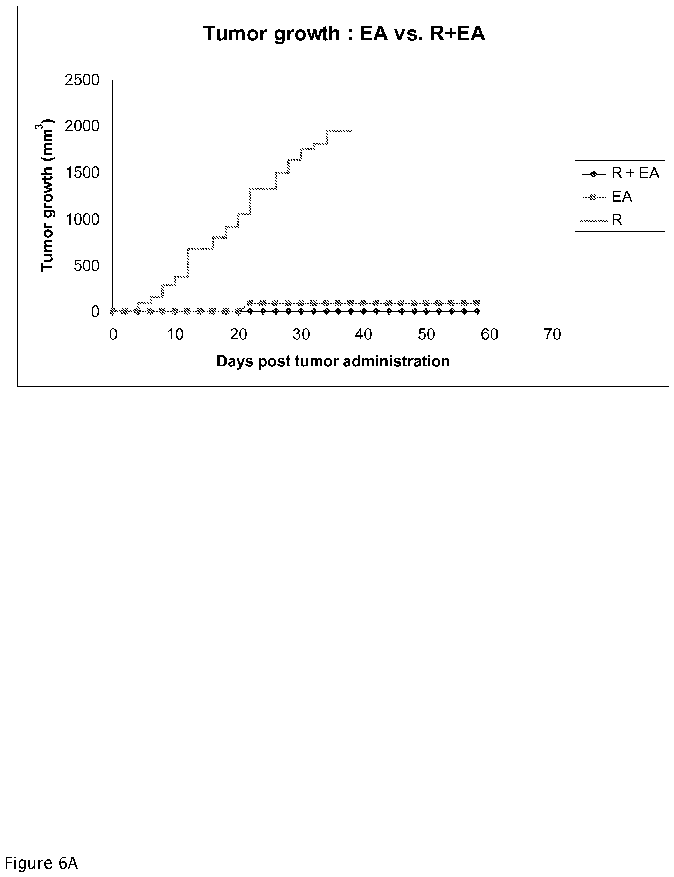

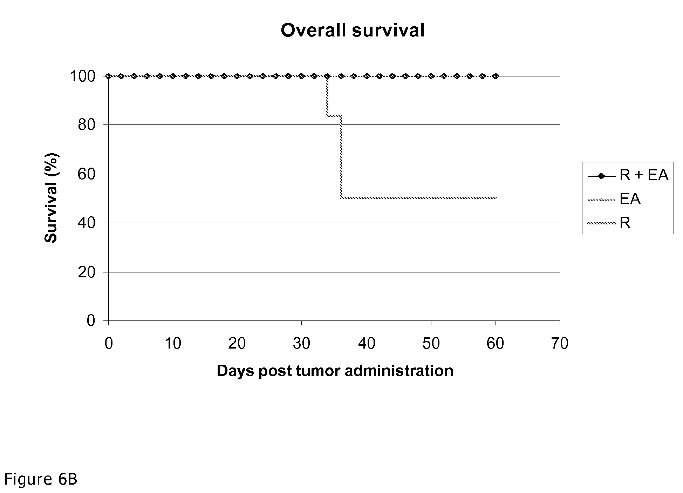

The effect of lenalidomide, EA and of the combination of lenalidomide and EA was tested in vivo. BALB/c mice were injected with 5x105 MPC11 myeloma cells subcutaneously. Tumor growth as well as overall survival were significantly reduced (p<0.001, respectively) in mice treated with EA or EA plus lenalidomide as compared to mice treated with lenalidomide alone (Figures 6A, B).

### Discussion

The Wnt signaling pathway has been shown to play a critical role in the early phases of B lymphocyte development. Multiple myeloma (MM) cells, but not cells from healthy donors and patients with monoclonal gammopathy of undetermined significance or other plasma cell dyscrasias involving bone marrow express the Wnt-signaling antagonist DKK1¹⁸. It has been reported that secretion of DKK1 by MM cells likely contributes to the formation of osteolytic lesions in this disease by inhibiting Wnt signaling: the latter being essential for osteoblast differentiation and survival. Changes of DKK1 expression in MM cells could be traced through disturbances in the JNK signaling cascade which is differentially modulated by oxidative stress and interactions between MM cells with osteoclasts, in vitro. Despite its role as a tumor suppressor and mediator of apoptosis in other cell types, including osteoblasts, the data indicate that DKK1 - a stress-responsive gene in MM - does not mediate apoptotic signaling, is not activated by TP53, and its forced over expression could not inhibit cell growth or sensitize MM cells towards apoptosis, following treatment with either thalidomide or lenalidomide. Therefore, specific strategies may be beneficial to modulate persistent activation of the JNK pathway in preventing disease progression and treating myeloma associated bone disease by inhibiting DKK1 expression¹⁸.

Many hypotheses have been proposed to explain the molecular mechanism of thalidomide's teratogenicity, in particular regarding limb defects. Most experimental evidence in vivo has been provided for a model suggesting the generation of oxidative stress by thalidomide with subsequent down regulation of both Wnt and Akt survival pathways. In addition, transcription factors Tbx5 and Sall4 are involved in thalidomide induced molecular pathology¹⁹.

Since thalidomide down regulates the Wnt pathway and is used for treatment of myeloma patients, testing the combination of thalidomide and Wnt inhibitors became of particular interest. We used EA, a drug that has recently been shown to induce apoptosis and down regulate the beta catenin expression in lymphoma cells. Most interestingly, we could demonstrate a synergistic effect of the combination of thalidomide and EA in myeloma cells.

In addition to thalidomide, nowadays patients with myeloma are treated more frequently with the novel agents bortezomib and lenalidomide. Therefore, we tested these drugs in myeloma cells in combination with the Wnt inhibitor EA. Our research demonstrates a synergistic effect of the combination of lenalidomide and EA in myeloma cells. However, we failed to demonstrate such an effect for the combination of bortezomib and the Wnt inhibitors. That might be explained by the molecular relationship of thalidomide and lenalidomide in contrast to bortezomib which belongs to a completely different class of drugs. In the lymphoma cell lines SU-DHL4 and LAM-53 the wnt inhibitor EA induced apoptosis through down regulation of beta catenin, an important molecule within the wnt pathway. However, in myeloma, when exposed to EA, β-catenin expression increased in RPMI cells and stayed similar in U266 cells. At present is unclear why EA acts so differently on lymphoma and on myeloma cells.

Recently, we also could show that EA is efficacious in primary cultures derived from patients with chronic lymphocytic leukemia¹⁶. EA was identified as a Wnt inhibitor using a cell-based Wnt reporter assay. *In vitro* assays further confirmed the inhibitory effect of EA on Wnt/β-catenin signaling. Cell viability assays showed that EA selectively induced cell death in primary CLL cells. Exposure of CLL cells to EA decreased the expression of Wnt/β-catenin target genes, including LEF-1, cyclin D1 and fibronectin. Immune co-precipitation experiments demonstrated that EA could directly bind to LEF-1 protein and destabilize the LEF-1/β-catenin complex. N-acetyl-L-cysteine (NAC), which can react with the a, β-unsaturated ketone in EA, but not other anti-oxidants, prevented the drug's inhibition of Wnt/β-catenin activation and its ability to induce apoptosis in CLL cells.

Our results are in accordance with a recent report of Sukhdeo et al. demonstrating that the canonical Wnt signaling pathway is activated in multiple myeloma through constitutively active beta catenin. Interestingly, we could demonstrate a synergistic effect of the combination of thalidomide as well as lenalidomide and Wnt inhibitors in myeloma cells. This observation might lead to new treatment options in patients with multiple myeloma.

**Table I**

| **Cell line** | **IC₅₀ thalidomide** | **IC₅₀ lenalidomide** | **IC₅₀ EA** |
|---|---|---|---|
| OPM-2 | 53 µM | 13 µM | 22 µM |
| U266 | 300 µM | 34 µM | 60 µM |
| RPMI 8226 | > 400 µM | 2 µM | 8 µM |
| Raji | > 400 µM | > 1000 µM | 33 µM |
| OCI-Ly8 LAM53 | > 400 µM | > 1000 µM | 57 µM |
| SU-DHL 4 | > 400 µM | > 1000 µM | 58 µM |
| PBMC | > 400 µM | > 400 µM | > 400 µM |
| PBL | > 400 µM | > 200 µM | 10 µM |

**Table I:** The effect of thalidomide, lenalidomide and EA on myeloma (OPM-2, U266 and RPMI 8226) and lymphoma (OCI-Ly8 LAM53, SU-DHL-4 and Raji) cell lines was assayed. PBMC and PBL derived from healthy individuals were used as normal controls. 1x10⁵ cells were cultured with each compound using various concentrations for three days. Then cell viability was measured by DIOC₆ staining by flow cytometry. Results represent data from two to four separate experiments each.

### Figure legends

**Figure 1****. Effect of thalidomide in combination with EA on viability of the U266 (A), RPMI (B) and OPM-2 (C) myeloma cells.** 1x10⁵ cells were cultured with different compounds for three days. The concentration of thalidomide was U266 160µM for U266, RPMI 20µM for RPMI, and OPM 40µM for OPM cells. Then cell viability was measured by DIOC₆ staining in flow cytometry. Results represent data from three, two and five separate experiments, respectively. Separate U266 n=3, RPMI n=2, OPM n=5 experiments. Data are shown as mean ± SD.
**Figure 2****. Effect of lenalidomide in combination with EA on viability of OPM-2 (A) and RPMI (B) myeloma cells.** 1x10⁵ OPM cells were cultured with the different compounds for three days. The concentration of lenalidomide was 12µM for OPM and 80µM for RPMI cells. Then cell viability was measured by DIOC₆ staining in flow cytometry. Results represent data from eight and five OPM n=8, RPMI n=5 separate experiments, respectively. Data are shown as mean ± SD.
**Figure 3****. Effect of EA on the Wnt/beta catenin pathway in lymphoma cells.** (A) Flow cytometric results. (B) We analyzed the effect of EA on the Wnt/beta catenin pathway by Western blot.
**Figure 4A****. Western Blot analysis for the detection of β-catenin in myeloma cell lines under normal conditions.** The myeloma cell lines RPMI 8226, KMS-18 and U266 were analyzed according to their β-catenin expression.
**Figure 4B****. Effect of EA on β-catenin expression by Western blot analysis.** KMS-18 cells were treated with EA for 24h, cells were lysed and then Western blot analysis was performed for β-catenin. β-actin immunoblotting served as the loading control. As primary antibodies purified mouse anti-β-catenin and β-actin were used at a dilution of 1:1000 and as secondary antibody served goat anti-mouse IgG-HRP at a dilution of 1:5000. Finally, ECL reagent was utilized for detection.
**Figure 4C****. Effect of EA on β-catenin expression by Western blot analysis.** RPMI 8226 cells were treated with EA for 24h, cells were lysed and then Western blot analysis was performed for β-catenin. β-actin immunoblotting served as the loading control. As primary antibodies Purified mouse anti-β-catenin and β-actin were used at a dilution of 1:1000 and as secondary antibody served goat anti-mouse IgG-HRP at a dilution of 1:5000. Finally, ECL reagent was utilized for detection.
**Figure 5****. Effect of EA on viability of myeloma primary cells.** Primary myeloma cells derived from bone marrow from patients with multiple myeloma were incubated for three days with EA Then cell viability was measured by DIOC₆ staining in flow cytometry. Results represent data from two separate experiments. Data are shown as mean.
**Figure 6****. In vivo effect of lenalidomide, EA and of the combination of lenalidomide (R) and EA.** The effect of lenalidomide, EA and of the combination of lenalidomide and EA was tested in vivo. BALB/c mice were injected with 5x10⁵ MPC11 myeloma cells subcutaneously. (A) Tumor growth and (B) overall survival were measured. Six animals were assayed per group.

### Increased Efficacy of Lenalidomide And Thalidomide By Addition Of Ciclopirox olamine Targeting The Wnt/Beta-catenin Pathway

Major progress has been achieved in the treatment of multiple myeloma by the introduction of novel agents like thalidomide, lenalidomide and bortezomib. Nevertheless, myeloma remains an incurable disease. In newly diagnosed patients, the combination of lenalidomide and dexamethasone has a response rate of 91%.

Several groups have shown that the Wnt/beta catenin pathway plays an important role in the regulation of cell proliferation, differentiation and apoptosis [1-3]. Aberrant activation of the Wnt signaling pathway has major oncogenic effects [4-7]. In the canonical Wnt pathway, the secreted Wnt proteins bind to a receptor complex, consisting of a member of the Frizzled (Fzd) family, and the low-density lipoprotein-receptor-related proteins (LRP) 5 or LRP6. Subsequently the cytoplasmic adaptor protein disheveled (Dvl) is phosphorylated and inhibits glycogen synthase kinase (GSK)-3β activity through its association with axin. Unphosphorylated β-catenin accumulates in the cytoplasm and translocates into the nucleus, where it interacts with T cell (TCF) and lymphoid-enhancing (LEF) factors to activate transcription of Wnt target genes [4,5,8]. In addition, it has been demonstrated that the Wnt pathway is activated in lymphoma. Therefore, the Wnt/beta catenin signaling molecules are attractive candidates for development of targeted approaches in lymphoma treatment.

In more recent work, our group confirmed that the diuretic agent ethacryanic acid (EA) and the antifungal agent ciclopirox olamine (cic) inhibit Wnt/beta catenin signaling [9]. Ciclopirox olamine (cic) is a synthetic antifungal agent used topically for the treatment of yeast infections in humans and is degraded by glucoronidation [11]. It serves as a chelator of polyvalent metal cations (e.g. Fe³⁺ and Al³⁺) resulting in the inhibition of metal depending enzymes, occurring in the metabolism of the cell. Furthermore, it blocks the cell cycle near the G1/S phase boundary [15].

For all of these reasons, we tested the effect of CIC on myeloma cells in addition to the commonly used therapeutic drugs.

### Design and Methods

### Cell lines and culture conditions

The lymphoma cell lines LAM-53, SU-DHL-4, Daudi and Raji, as well as the myeloma cell lines OPM-2, RPMI-8226 and U-266 (all obtained from DSMZ, Collection of Microorganisms and Cell Culture, Braunschweig, Germany) were cultured in RPMI-1640 Medium consisting of 10 % haet-inactivated fetal calf serum (FCS, Invitrogen, Karlsruhe, Germany), 2.5 % 1 M HEPES, and 1x penicillin/streptomycin (all from PAA Laboratories GmbH, Cölbe, Germany). Cells were cultured at a density of 3.3 x 10⁵ cells ml⁻¹ and incubated at 37°C with 5 % CO₂ and 95% humidity.

MPC11 (DMSZ, Braunschweig, Germany) is a murine plasmocytoma cell line derived from the Balb/c strain expressing IgG2b. Cells were cultured in RPMI 1640 medium (PAA Laboratories GmbH, Austria) supplemented with 5% fetal calf serum (FCS), 2 mM glutamine (both from PAA, Cölbe, Germany), 100 U/ml penicillin / 100 U/ml streptomycin (both from Seromed, Jülich, Germany) at 37°C in a humidified 5%CO₂ atmosphere.

### Human samples

Peripheral blood mononuclear cells (PBMC) and PBL were isolated from blood samples of healthy volunteers by Ficoll density gradient centrifugation (Lymphoprep, Nycomed, Oslo, Norway). In addition, bone marrow samples from patients with myeloma were obtained. Ethics approval had been obtained according to the guidelines of our institution.

### Drugs and chemical reagents

In our experiments the following drugs were used: ciclopirox olamine (CIC; Sigma-Aldrich GmbH, Seelze, Germany), doxorubicin (Cellpharm GmbH, Germany), rituximab (Roche Pharma AG, Grenzach Wyhlen, Germany), bortezomib (Janssen Cilag, Neuss, Germany), thalidomide (Grünenthal Pharma GmbH, Aachen, Germany). Lenalidomide was obtained from Celgene (Munich, Germany). All drugs were tested at various concentrations for 24 - 72 hours.

### DiOC₆ and PI-staining

Experiments were performed as reported recently [17]. 1x10⁵ Cells were cultured in 3 ml Medium in 6-well plates. CIC was dissolved in DMSO, and added in an optimized concentration of 30 µM (CIC) alone or in combination with the therapeutic agents at various concentrations for three days. The apoptosis assay was performed with 3'3-dihexyloxacarbocyanine iodide (DiOC₆) detecting mitochondrial membrane potential in viable cells, and propidium iodide (PI) which binds to DNA in necrotic cells, measured by a fluorescence-activated cell sorter (FACS).

For FACS analysis, 500µl staining solution containing 80 nM DiOC₆ in FACS buffer, consisting of deficient RPMI-medium with 0.5% bovine serum albumin (BSA), was mixed with equal volumes of the cell sample in a glass tube and incubated at 37°C for 15 min. After a washing step with PBS/BSA 1% the cells were re-suspended in 500µl PBS/BSA 1%. After addition of 5µl PI-solution (100µg/ml) the cells were analyzed by FACS. Using this assay, viable cells reveal high fluorescence intensity for DiOC₆ and a low expression for PI. In contrary, apoptotic cells show a low expression for DiOC₆ and also a low expression for PI (2). Finally, necrotic cells show a low expression for DiOC₆ and a high expression for PI.

A mean IC₅₀ value in myeloma cells was determined using the mean of the IC₅₀ results determined in OPM-2, U266 and RPMI 8226 cells.

### Isolation of peripheral blood mononuclear cells (PBMCs)

PBMCs were isolated from blood of healthy donors by Ficoll-Hypaque density gradient centrifugation. Blood from buffy coats was mixed 1:2 with PBS/1% BSA (both PAA, Cölbe, Germany) and used for a ficoll gradient (LymphoPrep, PAA, Cölbe, Germany). After the centrifugation at 800 g for 30 minutes, the leukocyte layer was removed and transferred to new tubes. Subsequently, these cells were washed 3 times with PBS/1%BSA and re-suspended in fresh medium, consisting of RPMI medium (PAA, Cölbe, Germany) with 10% fetal calf serum (Invitrogen, Karlsruhe, Germany), 2.5% hepes buffer solution (PAA, Cölbe, Germany), and 1% penicillin/streptomycin (PAA, Cölbe, Germany).

### Bone marrow samples

Bone marrow samples from patients with myeloma were taken after informed consent, single cell suspensions generated and cells ficolled. Cells were incubated with or without CIC for three days and measured for viability.

### Western blot

We analyzed the effect of CIC on the Wnt/beta catenin pathway by Western blot. Western blot was performed as described recently [16].

### Animal studies

All animal experiments were done at least in duplicate with groups of 6 BALB/c mice (Charles River, Sulzfeld, Germany). 5x10⁵ MCP11 murine myeloma cells were injected per mouse subcutaneously into Balb/c mice. Mice were treated orally with 450µg/day CIC and 750 µg lenalidomide/day, respectively. Overall survival and tumor growth were measured. Tumor volume was calculated as follows: volume = length x width² x 0,52. Animals were killed when tumor volume reached 2000 mm³,

### Statistical analysis

For statistical analysis, the numbers of the results comprising the relative viability are expressed as the mean ± standard error of the mean (SEM). Different sample sizes (n) were chosen for different cell lines. Student's t test was used for statistical analysis. A p value below 0.05 was considered significant. Statistical survival analyses were performed with the software GraphPad InStat, Version 3.0.0 (GraphPad Software, San Diego, CA), applied the Mann-Whitney test (non-paired, non-parametric).

### Results

### Inhibition of Wnt/beta catenin signaling by CIC

In our recent research we used a 96-well plate-based TOPflash reporter system to screen the Gen-plus drug library (Microsource) that contained 960 compounds. The screen identified CIC and cic as Wnt/beta catenin inhibitors [16]. Since the canonical Wnt signaling pathway is activated in lymphoma and myeloma cells we further investigated if CIC could induce apoptosis and decrease viability of lymphoma and myeloma cell lines, respectively.

### Titration of CIC

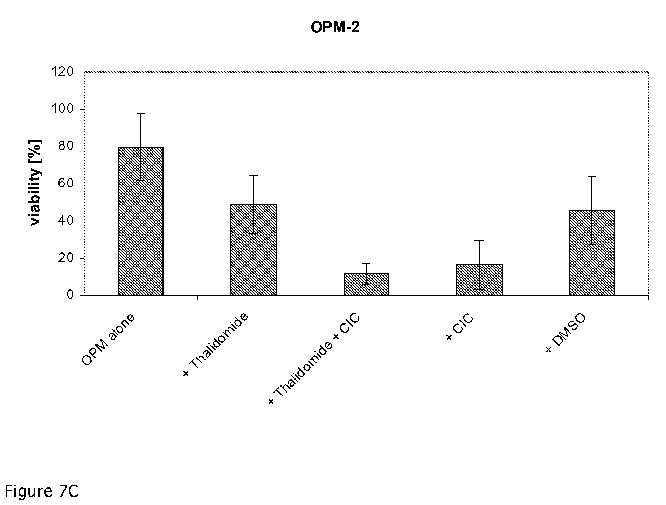

As a first step, we determined the optimal concentration of CIC in various lymphoma and myeloma cell lines. Subsequently, we determined the optimal concentration of CIC in PBMCs derived from healthy individuals in order to check for toxicity. Determination of the optimal concentration of CIC revealed that 30 µM CIC was the most effective concentration to initialize cell death in both lymphoma and myeloma cell lines, without significantly deteriorating viability of normal PBMCs (data not shown). The effect of DMSO as a toxic solvent was only observed in the myeloma cell line OPM-2 (Fig. 7C). As a control, peripheral blood mononuclear cells (PBMCs) were investigated by FACS analysis. A toxic effect affecting the cells induced a shift from viable, DiOC₆-positive cells to apoptotic, DiOC₆-negative cells.

### Viability of cell lines and PBMC after 72 hours of culture

Viability decreased slowly over time. After 72 h, IC50 values for the lymphoma cell lines SU-DHL-4, LAM-53 and Raji in the presence of CIC were 7uM, 2uM and 6uM, respectively. In contrast, IC50 in PBMC was above 400uM (Table II).

### Effect of DMSO on viability of cell lines

In contrast to PBMC and lymphoma cell lines, the myeloma cell line OPM-2 was highly sensitive towards DMSO and showed a decrease of relative viability to 66.8 +/- 2.3%.

### Effect of doxorubicin in combination with CIC on viability of lymphoma cell lines

In general, lymphoma patients are treated with doxorubicin and rituximab besides other drugs. Therefore, we tested these drugs in lymphoma cells in combination with CIC.

In Daudi and Raji cells, the addition of doxorubicin led to a significant decrease in viability of Daudi and Raji cells. However, the combination of CIC plus doxorubicin did not further decrease viability of Raji cells. Therefore, we would exclude a synergistic effect (data not shown).

### Effect of rituximab in combination with CIC on viability of lymphoma cell lines

In Daudi and Raji cells, the addition of rituximab led to significantly decreased viability of these cells. However, the combination of CIC plus rituximab did not further decrease viability of Daudi and Raji cells. Like with doxorubicin, we excluded a synergistic effect of this combination (data not shown).

### Effect of bortezomib in combination with CIC on viability of myeloma cell line OPM-2

Treatment of myeloma has changed in recent times. More and more patients are treated with novel drugs, such as bortezomib, lenalidomide and thalidomide - the latter already is known for quite some time. Therefore, we tested these drugs in OPM-2 myeloma cells in combination with CIC.

In OPM-2 cells, the addition of bortezomib led to a significant decrease in viability of OPM-2 myeloma cells. However, the combination of CIC plus bortezomib did not further decrease viability of OPM cells. We excluded a synergistic effect of this combination, as well (data not shown).

### Effect of thalidomide, lenalidomide and CIC on viability of myeloma cell lines

All three compounds significantly (p<0.05) decreased the viability of myeloma cell lines in vitro (Table II). A mean IC₅₀ value in myeloma cells was determined: Lenalidomide (16.3uM) and CIC (30uM) were more effective than thalidomide (>251uM; Table II).

In contrast the effect on normal PBMC and PBL was marginal in CIC and lenalidomide. Here, no effect was found for thalidomide (Table II).

### Effect of thalidomide, lenalidomide and CIC on viability of lymphoma cell lines

Only CIC significantly (p<0.05) decreased the viability of lymphoma cell lines in vitro (Table II). In contrast, lenalidomide and thalidomide showed no effect (Table II).

### Effect of thalidomide in combination with CIC on viability of myeloma cell lines

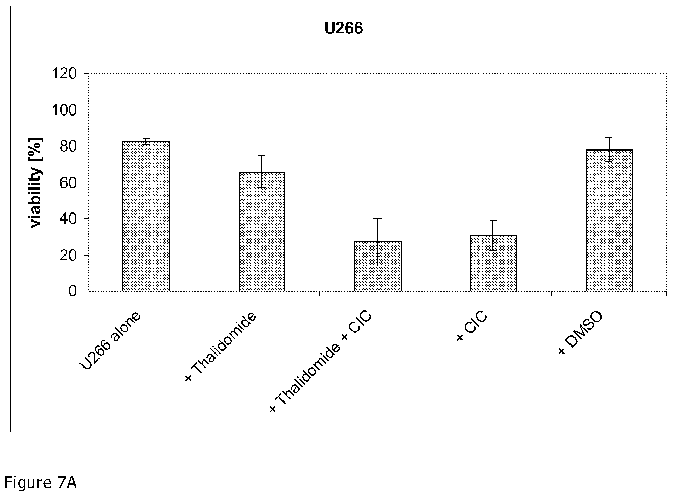

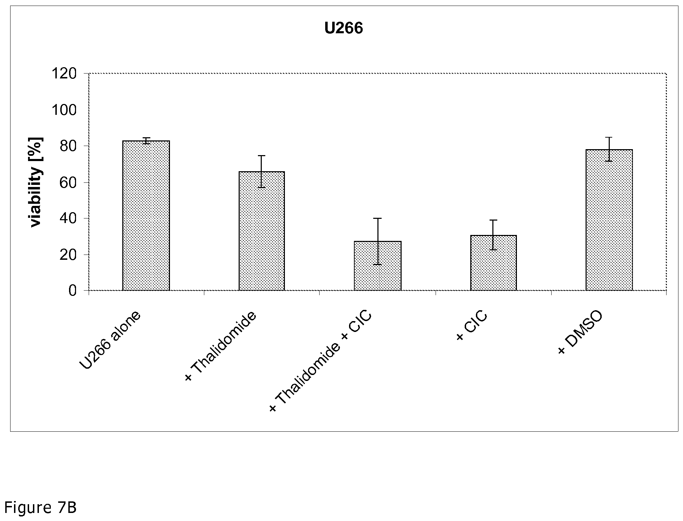

In OPM-2, U-266 and RPMI-8226 cells, the addition of thalidomide led to a significant decrease in viability of OPM-2, U-266 and RPMI-8226 myeloma cells. Interestingly, the combination of CIC plus thalidomide led to a further decrease of viability of OPM-2, U-266 and RPMI-8226 cells; suggesting a synergistic effect for this combination (Fig. 7).

### Effect of lenalidomide in combination with CIC on viability of myeloma cell lines

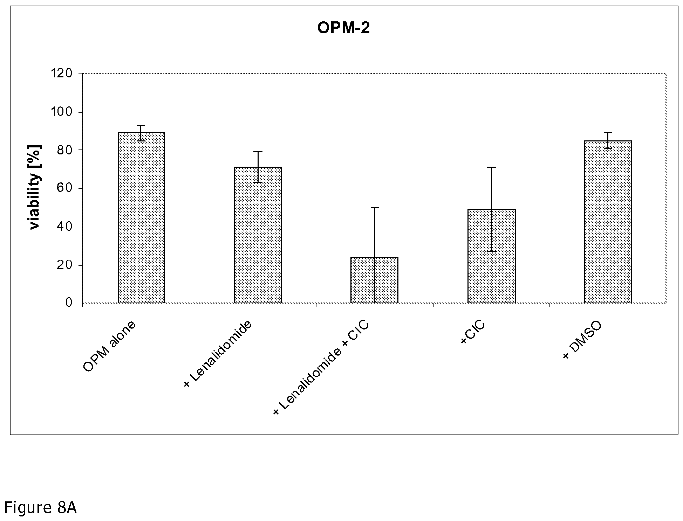

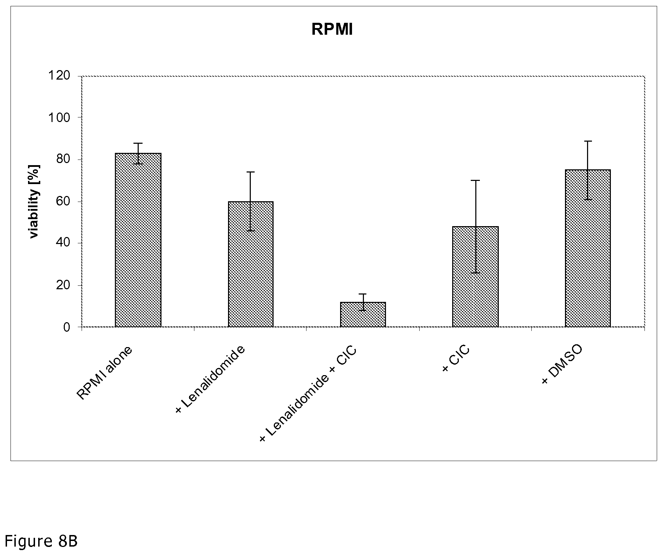

In OPM-2 and RPMI-8226 cells, the addition of lenalidomide led to a significant decrease in viability of OPM-2 and RPMI-8226 myeloma cells. Like with thalidomide, the addition of lenalidomide to CIC led to further decreased viability of both OPM-2 and RPMI-8226 cells. These results indicate a synergistic effect for this combination (Fig. 8).

### Effect of CIC on the Wnt/beta catenin pathway in lymphoma cells

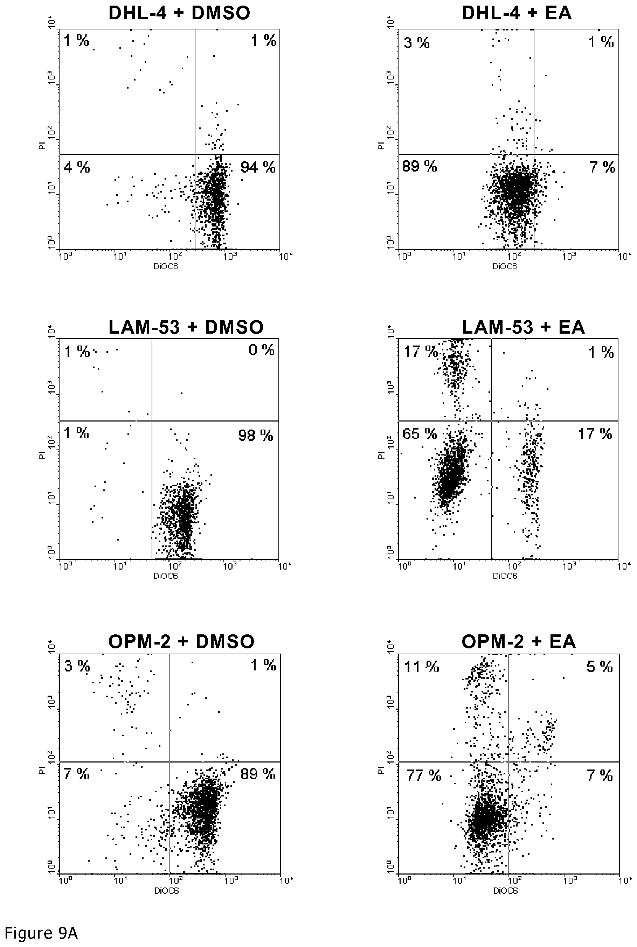

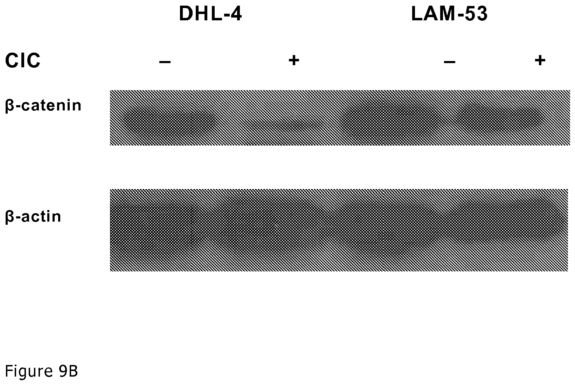

As a next step, we analyzed the effect of CIC on the Wnt/beta catenin pathway. The myeloma cell line OPM-2 did not express enough beta catenin compared to lymphoma cell lines. However, we could show that beta catenin is down regulated when CIC is added to lymphoma cells (Fig. 9).

### Effect of CIC on the Wnt/beta catenin pathway in myeloma cells

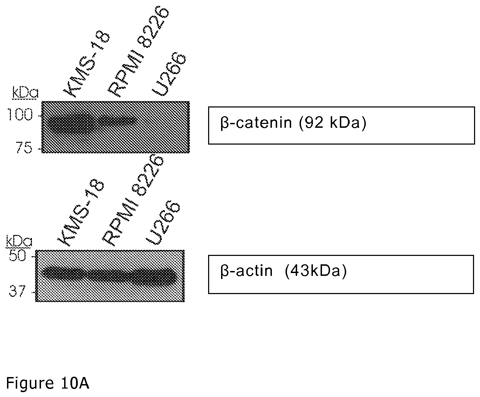

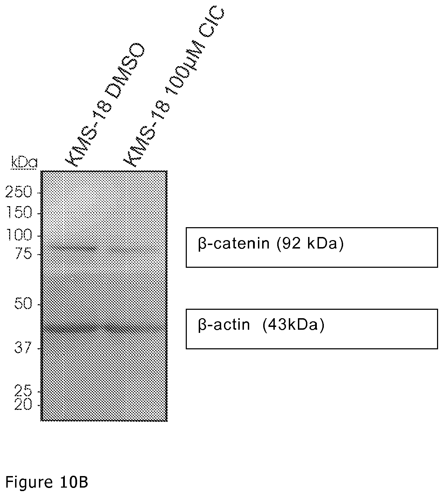

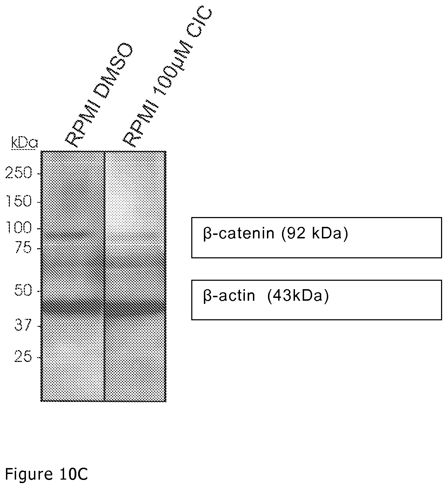

The effect of CIC on β-catenin expression in myeloma cells was tested. KMS18 and RPMI cells were found to be β-catenin positive in contrast to U266 cells being negative (Figure 10A). When exposed to CIC, β-catenin expression decreased in KMS-18 cells (Figure 10B) and slightly decreased in RPMI 8226 cells (Figure 10C).

### Effect of CIC on viability of myeloma primary cells

Preliminary data suggest that CIC inhibits the growth of primary myeloma cells derived from bone marrow from patients with multiple myeloma (Data not shown).

### Effect of lenalidomide, CIC and of the combination of lenalidomide and CIC in vivo

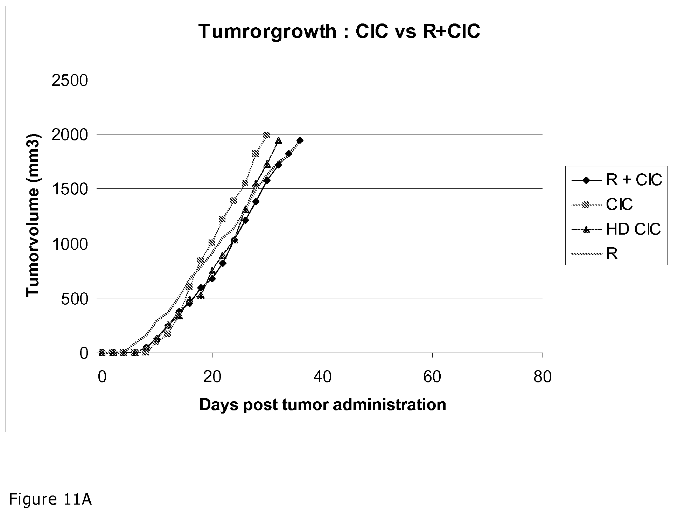

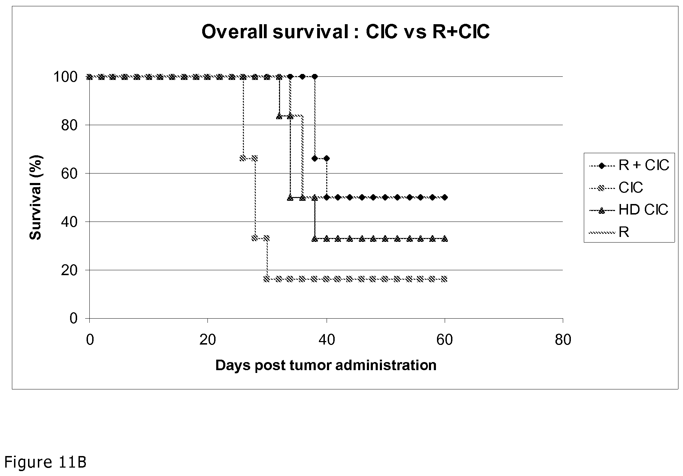

The effect of lenalidomide, CIC and of the combination of lenalidomide and CIC was tested in vivo BALB/c mice were injected with 5x105 MPC11 myeloma cells subcutaneously. Tumor growth as well as overall survival were significantly reduced in mice treated with CIC or CIC plus lenalidomide as compared to control mice (Fig. 11A and 11B).

### Discussion

The Wnt signaling pathway has been shown to play a critical role in the early phases of B lymphocyte development. Multiple myeloma (MM) cells, but not cells from healthy donors and patients with monoclonal gammopathy of undetermined significance or other plasma cell dyscrasias involving bone marrow express the Wnt-signaling antagonist DKK1 [18]. It has been reported that secretion of DKK1 by MM cells likely contributes to the formation of osteolytic lesions in this disease by inhibiting Wnt signaling: the latter being essential for osteoblast differentiation and survival. Changes of DKK1 expression in MM cells could be traced through disturbances in the JNK signaling cascade which is differentially modulated by oxidative stress and interactions between MM cells with osteoclasts, in vitro. Despite its role as a tumor suppressor and mediator of apoptosis in other cell types, including osteoblasts, the data indicate that DKK1 - a stress-responsive gene in MM - does not mediate apoptotic signaling, is not activated by TP53, and its forced over expression could not inhibit cell growth or sensitize MM cells towards apoptosis, following treatment with either thalidomide or lenalidomide. Therefore, specific strategies may be beneficial to modulate persistent activation of the JNK pathway in preventing disease progression and treating myeloma associated bone disease by inhibiting DKK1 expression [18].

Many hypotheses have been proposed to explain the molecular mechanism of thalidomide's teratogenicity, in particular regarding limb defects. Most experimental evidence in vivo has been provided for a model suggesting the generation of oxidative stress by thalidomide with subsequent down regulation of both Wnt and Akt survival pathways. In addition, transcription factors Tbx5 and Sall4 are involved in thalidomide induced molecular pathology [19].

Since thalidomide down regulates the Wnt pathway and is used for treatment of myeloma patients, testing the combination of thalidomide and Wnt inhibitors became of particular interest. We used CIC, a drug that has recently been shown to induce apoptosis and down regulate the beta catenin expression in lymphoma cells. Most interestingly, we could demonstrate a synergistic effect of the combination of thalidomide and CIC in myeloma cells.

In addition to thalidomide, nowadays patients with myeloma are treated more frequently with the novel agents bortezomib and lenalidomide. Therefore, we tested these drugs in myeloma cells in combination with the Wnt inhibitor CIC. Our research demonstrates a synergistic effect of the combination of lenalidomide and CIC in myeloma cells. However, we failed to demonstrate such an effect for the combination of bortezomib and the Wnt inhibitors. That might be explained by the molecular relationship of thalidomide and lenalidomide in contrast to bortezomib which belongs to a completely different class of drugs. In the lymphoma cell lines SU-DHL4 and LAM-53 the wnt inhibitor CIC induce apoptosis through down regulation of beta catenin, an important molecule within the wnt pathway.

In conclusion, our results reveal a significant induction of apoptosis by CIC in both lymphoma and myeloma cells. Combined with our previous results, our data suggest that CIC can inhibit Wnt/beta catenin signaling in both lymphoma and myeloma cell lines.
Recently, we also could show that a different drug inhibiting the wnt pathway, EA, is efficacious in primary cultures derived from patients with chronic lymphocytic leukemia [16]. CIC and EA were identified as a Wnt inhibitor using a cell-based Wnt reporter assay. *In vitro* assays further confirmed the inhibitory effect of EA and CIC on Wnt/β-catenin signaling. Cell viability assays showed that CIC and EA selectively induced cell death in primary CLL cells. Exposure of CLL cells to EA decreased the expression of Wnt/β-catenin target genes, including LEF-1, cyclin D1 and fibronectin. Immune co-precipitation experiments demonstrated that EA could directly bind to LEF-1 protein and destabilize the LEF-1/β-catenin complex. N-acetyl-L-cysteine (NAC), which can react with the a, β-unsaturated ketone in EA, but not other anti-oxidants, prevented the drug's inhibition of Wnt/β-catenin activation and its ability to induce apoptosis in CLL cells.
Our results are in accordance with a recent report of Sukhdeo et al. demonstrating that the canonical Wnt signaling pathway is activated in multiple myeloma through constitutively active beta catenin. Interestingly, we could demonstrate a synergistic effect of the combination of thalidomide as well as lenalidomide and Wnt inhibitors in myeloma cells. This observation might lead to new treatment options in patients with multiple myeloma.

**Table II**

| **Cell line** | **IC₅₀ thalidomide** | **IC₅₀ lenalidomide** | **IC₅₀ CIC** |
|---|---|---|---|
| OPM-2 | 53 µM | 13 µM | 5 µM |
| U266 | 300 µM | 34 µM | 6 µM |
| RPMI 8226 | > 400 µM | 2 µM | 6 µM |
| Raji | > 400 µM | > 1000 µM | 6 µM |
| OCI-Ly8 LAM 53 | > 400 µM | > 1000 µM | 2 µM |
| SU-DHL 4 | > 400 µM | > 1000 µM | 7 µM |
| PBMC | > 400 µM | > 400 µM | > 400 µM |
| PBL | > 400 µM | > 200 µM | 9 µM |

**Table II:** The effect of thalidomide, lenalidomide and CIC on myeloma (OPM-2, U266 and RPMI 8226) and lymphoma (LAM53, SU-DHL-4 and Raji) cell lines was assayed. PBMC and PBL derived from healthy individuals were used as normal controls. 1x10⁵ cells were cultured with each compound using various concentrations for three days. Then cell viability was measured by DIOC₆ staining by flow cytometry. Results represent data from 2 to 4 separate experiments each.

### Figure legends

**Figure 7****. Effect of thalidomide in combination with CIC on viability of the U266 (A), RPMI (B) and OPM-2 (C) myeloma cells.** 1x10⁵ cells were cultured with different compounds for three days. The concentration of thalidomide was U266 160µM for U266, RPMI 20µM for RPMI, and OPM 40µM for OPM cells. Then cell viability was measured by DIOC₆ staining in flow cytometry. Results represent data from three, two and five separate experiments, respectively. Separate U266 n=3, RPMI n=2, OPM n=5 experiments. Data are shown as mean ± SD.
**Figure 8****. Effect of lenalidomide in combination with CIC on viability of OPM-2 (A) and RPMI (B) myeloma cells.** 1x10⁵ OPM cells were cultured with the different compounds for three days. The concentration of lenalidomide was 12µM for OPM and 80µM for RPMI cells. Then cell viability was measured by DIOC₆ staining in flow cytometry. Results represent data from eight and five OPM n=8, RPMI n=5 separate experiments, respectively. Data are shown as mean ± SD.
**Figure 9****. Effect of CIC on the Wnt/beta catenin pathway.** (A) Flow cytometric results. (B) We analyzed the effect of CIC on the Wnt/beta catenin pathway by Western blot.
**Figure 10A****. Western Blot analysis for the detection of β-catenin in myeloma cell lines under normal conditions.** The myeloma cell lines RPMI 8226, KMS-18 and U266 were analyzed according to their β-catenin expression.
**Figure 10B****. Effect of CIC on β-catenin expression by Western blot analysis.** KMS-18 cells were treated with CIC for 24h, cells were lysed and then Western blot analysis was performed for β-catenin. β-actin immunoblotting served as the loading control. As primary antibodies purified mouse anti-β-catenin and β-actin were used at a dilution of 1:1000 and as secondary antibody served goat anti-mouse IgG-HRP in a dilution of 1:5000. Finally, ECL reagent was utilized for detection.
**Figure 10C****. Effect of CIC on β-catenin expression by Western blot analysis.** RPMI 8226 cells were treated with CIC for 24h, cells were lysed and then Western blot analysis was performed for β-catenin. β-actin immunoblotting served as the loading control. As primary antibodies Purified mouse anti-β-catenin and β-actin were used at a dilution of 1:1000 as secondary antibody served goat anti-mouse IgG-HRP at a dilution of 1:5000. Finally, ECL reagent was utilized for detection.
**Figure 11****. In vivo effect of lenalidomide, CIC and of the combination of lenalidomide (R) and CIC.** (A) Tumor growth and (B) overall survival were measured. Six animals were assayed per group. HD CIC.

### Increased In vivo Efficacy Of Lenalidomide by Addition Of Piroctone olamine

Major progress has been achieved in the treatment of multiple myeloma by the introduction of novel agents like thalidomide, lenalidomide and bortezomib. Nevertheless, multiple myeloma remains an incurable disease. In newly diagnosed patients, the combination of lenalidomide and dexamethasone has a response rate of 91%.

Several groups have shown that the Wnt/beta catenin pathway plays an important role in the regulation of cell proliferation, differentiation and apoptosis [1-3]. Aberrant activation of the Wnt signaling pathway has major oncogenic effects [4-7]. In the canonical Wnt pathway, the secreted Wnt proteins bind to a receptor complex, consisting of a member of the Frizzled (Fzd) family and the low-density lipoprotein-receptor-related proteins (LRP) 5 or LRP6. Subsequently, the cytoplasmic adaptor protein disheveled (Dvl) is phosphorylated and inhibits glycogen synthase kinase (GSK)-3β activity through its association with axin. Unphosphorylated β-catenin accumulates in the cytoplasm and translocates into the nucleus, where it interacts with T cell (TCF) and lymphoid-enhancing (LEF) factors to activate transcription of Wnt target genes [4,5,8]. In addition, it has been demonstrated that the Wnt pathway is activated in lymphoma. Thus, the Wnt/beta catenin signaling molecules are attractive candidates for development of targeted approaches in lymphoma treatment.

In a more recent work, our group confirmed that the antifungal agent ciclopirox olamine (CIC) inhibits Wnt/beta catenin signaling [9]. Ciclopirox olamine (CIC) is a synthetic antifungal agent used topically for the treatment of yeast infections in humans and is degraded by glucoronidation [11]. It serves as a chelator of polyvalent metal cations (e.g. Fe³⁺ and Al³⁺) resulting in the inhibition of metal depending enzymes, occurring in the metabolism of the cell. Furthermore, it blocks the cell cycle near the G1/S phase boundary [15].

Piroctone olamine (PO), an ethanolamine salt of the hydroxamic acid derivative piroctone, is a pyridone derivate as is CIC. PO was first synthesized by Schwarzkopf - Henkel, Düsseldorf/Germany in 1979. It is a component of many cosmetic products for scall therapy and is known to have bactericidal effects on grampositive and gramnegative bacteriae as well as fungicidial effects. The agent penetrates the cell membrane and forms complexes with iron (III) ions inhibiting energy metabolism in mitochondria. In addition, it has been described as a new active collagenase inhibitor [20].

In this study, we investigated the effect of piroctone olamine (PO) on multiple myeloma and lymphoma cells in vitro and in vivo in a murine myeloma model.

### Methods

### Cell lines and culture conditions

The lymphoma cell lines LAM-53, SU-DHL-4, Daudi and Raji, as well as the myeloma cell lines OPM-2, RPMI-8226 and U-266 (all obtained from DSMZ, Collection of Microorganisms and Cell Culture, Braunschweig, Germany) were cultured in RPMI-1640 Medium consisting of 10 % heat-inactivated fetal calf serum (FCS, Invitrogen, Karlsruhe, Germany), 2.5 % 1 M HEPES, and 1x penicillin/streptomycin (all from PAA Laboratories GmbH, Cölbe, Germany). Cells were cultured at a density of 3.3 x 10⁵ cells ml⁻¹ and incubated at 37°C with 5 % CO₂ and 95% humidity.

MPC11 (DMSZ, Braunschweig, Germany) is a murine plasmocytoma cell line derived from the Balb/c strain expressing IgG2b. Cells were cultured in RPMI 1640 medium (PAA Laboratories GmbH, Austria) supplemented with 5% fetal calf serum (FCS), 2 mM glutamine (both from PAA, Cölbe, Germany), 100 U/ml penicillin / 100 U/ml streptomycin (both from Seromed, Jülich, Germany) at 37°C in a humidified 5%CO₂ atmosphere.

### Human samples

Peripheral blood mononuclear cells (PBMC) and PBL were isolated from blood samples of healthy volunteers by Ficoll density gradient centrifugation (Lymphoprep, Nycomed, Oslo, Norway). In addition, bone marrow samples from patients with myeloma were obtained. Ethics approval had been obtained according to the guidelines of our institution.

### Drugs and chemical reagents

In our experiments the following drugs were used: Thalidomide (Grünenthal Pharma GmbH, Aachen, Germany), lenalidomide was obtained from Celgene (Munich, Germany), and piroctone olamine (PO) from Spinnrad, Bonn, Germany. All drugs were tested at various concentrations for 24 - 72 hours. In addition, in this study piroctone olamine (PO, Spinnrad, Bonn, Germany) was applied orally.

### DiOC₆ and PI-staining

1x10⁵ Cells were cultured in 3 ml Medium in 6-well plates. The compounds EA and CIC were dissolved in DMSO, and added in optimized concentrations between 10 µM (CIC) and 30 µM (EA) alone or in combination with the therapeutic agents at various concentrations for three days. The apoptosis assay was performed with 3'3-dihexyloxacarbocyanine iodide (DiOC₆) detecting mitochondrial membrane potential in viable cells, and propidium iodide (PI) which binds to DNA in necrotic cells, measured by a fluorescence-activated cell sorter (FACS).

### Fluorescence-activated cell analysis

For FACS analysis, 500µl staining solution containing 80 nM DiOC₆ in FACS buffer, consisting of deficient RPMI-medium with 0.5% bovine serum albumin (BSA), was mixed with equal volumes of the cell sample in a glass tube and incubated at 37°C for 15 min. After a washing step with PBS/BSA 1% the cells were re-suspended in 500µl PBS/BSA 1%. After addition of 5µl PI-solution (100µg/ml) the cells were analyzed by FACS. Using this assay, viable cells reveal high fluorescence intensity for DiOC₆ and a low expression for PI. In contrary, apoptotic cells show a low expression for DiOC₆ and also a low expression for PI (2). Finally, necrotic cells show a low expression for DiOC₆ and a high expression for PI.

A mean IC₅₀ value in myeloma cells was determined using the mean of the IC₅₀ results determined in OPM-2, U266 and RPMI 8226 cells.

### Isolation of peripheral blood mononuclear cells (PBMCs)

PBMCs were isolated from blood of healthy donors by Ficoll-Hypaque density gradient centrifugation. Blood from buffy coats was mixed 1:2 with PBS/1% BSA (both PAA, Cölbe, Germany) and used for a ficoll gradient (LymphoPrep, PAA, Cölbe, Germany). After the centrifugation at 800 g for 30 minutes, the leukocyte layer was removed and transferred to new tubes. Subsequently, these cells were washed 3 times with PBS/1%BSA and re-suspended in fresh medium, consisting of RPMI medium (PAA, Cölbe, Germany) with 10% fetal calf serum (Invitrogen, Karlsruhe, Germany), 2.5% hepes buffer solution (PAA, Cölbe, Germany), and 1% penicillin/streptomycin (PAA, Cölbe, Germany).

### Bone marrow samples

Bone marrow samples from patients with myeloma were taken after informed consent, single cell suspensions generated and cells ficolled. Cells were incubated with or without CIC for three days and measured for viability.

### Western blot

We analyzed the effect of PO on the Wnt/beta catenin pathway by Western blot. Western blot was performed as described recently [21].

### Animal studies

All animal experiments were done at least in duplicate with groups of 6 BALB/c mice (Charles River, Sulzfeld, Germany). 5x10⁵ MCP11 murine myeloma cells were injected per mouse subcutaneously into Balb/c mice. Mice were treated orally with 450µg/day PO. Overall survival and tumor growth were measured. Tumor volume was calculated as follows: volume = length x width² x 0.52. Animals were killed when tumor volume reached 2000 mm³.

### Statistical analysis

For statistical analysis, the numbers of the results comprising the relative viability are expressed as the mean ± standard error of the mean (SEM). Different sample sizes (n) were chosen for different cell lines. Student's t test was used for statistical analysis. A p value below 0.05 was considered significant. Statistical survival analyses were performed with the software GraphPad InStat, Version 3.0.0 (GraphPad Software, San Diego, CA), applied the Mann-Whitney test (non-paired, non-parametric).

### Results

### Effect of piroctone olamine in vitro on viability of various cell lines

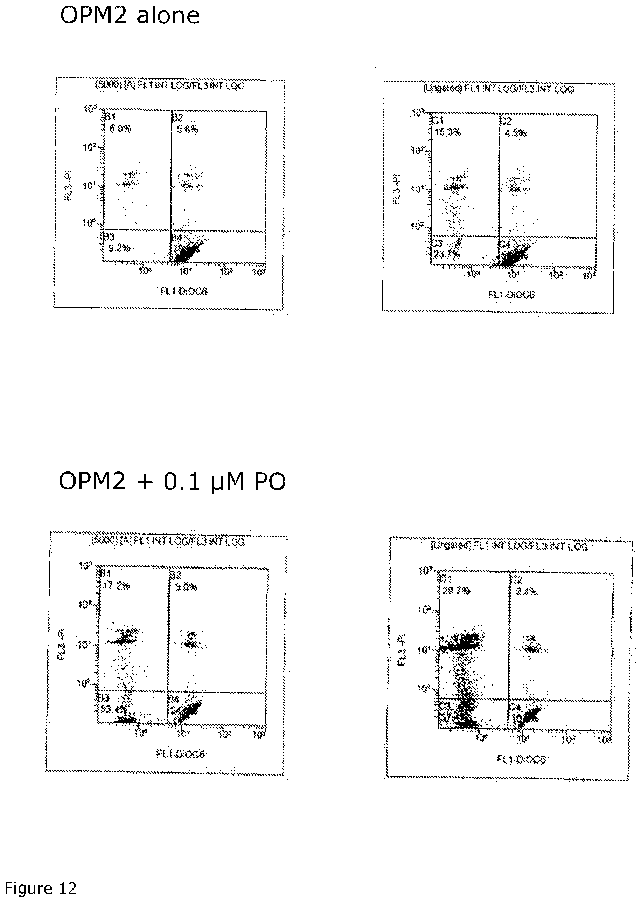

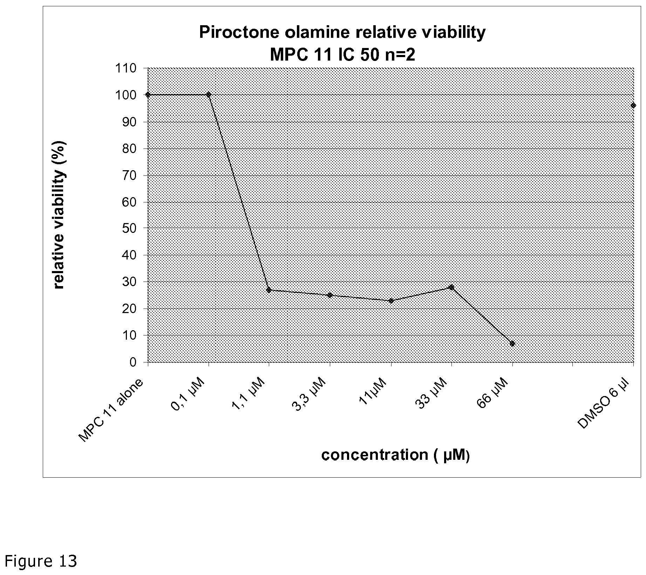

Piroctone olamine significantly decreased the viability of all myeloma cell lines in vitro (Table III, Fig. 12 and 13). Even small dosages of piroctone olamine were toxic, so the IC₅₀ mostly could be reached with less than 1µM of piroctone olamine for the human myeloma cell lines OPM-2 (0.7µM), U-266 (0.6µM), RPMI-8226 (0.6µM), KMS18 (0.6µM) and murine MPC-11 (0.8µM).

Similar results were obtained for human lymphoma cell lines Raji (0.6µM), Oci-Ly-Lam-53 (0.6µM) and SU-DHL-4 (0.7µM) (Table III).

In contrast, in PBLs (18µM), which were used as control cells, piroctone olamine was less efficient (Table III).

Cytotoxic activity was shown to be via induction of apoptosis (Figure 12).

### Effect of DMSO on viability of cell lines

In contrast to PBMC and lymphoma cell lines, the myeloma cell line OPM-2 was highly sensitive towards DMSO and showed a decrease of relative viability to 66.8 +/- 2.3%.

### Effect of thalidomide, lenalidomide and PO on viability of myeloma cell lines

All three compounds significantly (p<0.05) decreased the viability of myeloma cell lines in vitro (Table III, Fig. 13). A mean IC₅₀ value in myeloma cells was determined: Lenalidomide (16.3uM) and PO (0.6uM) were more effective than thalidomide (>251uM; Table III).

In contrast the effect on normal PBL was marginal in PO and lenalidomide. Here, no effect was found for thalidomide (Table III).

### Effect of thalidomide, lenalidomide and PO on viability of lymphoma cell lines

Only PO significantly (p<0.05) decreased the viability of lymphoma cell lines in vitro (Table III). In contrast, lenalidomide and thalidomide showed no effect (Table III).

### Effect of PO on the Wnt/beta catenin pathway in myeloma cells

The effect of EA on β-catenin expression in myeloma cells was tested. MPC11 cells were found to be β-catenin positive (data not shown).

### Effect of piroctone olamine on viability of myeloma primary cells

Preliminary data suggest that piroctone olamine inhibits the growth of primary myeloma cells derived from bone marrow from patients with multiple myeloma (data not shown).

### Effect of piroctone olamine on myeloma cells in vivo

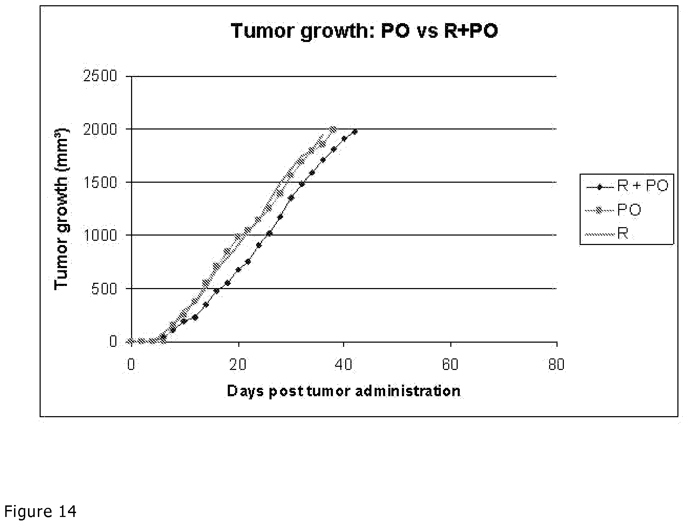

All animals in the control group developed a tumor larger than 2000 mm³ and therefore had to be sacrificed by day 22 (Fig. 14).

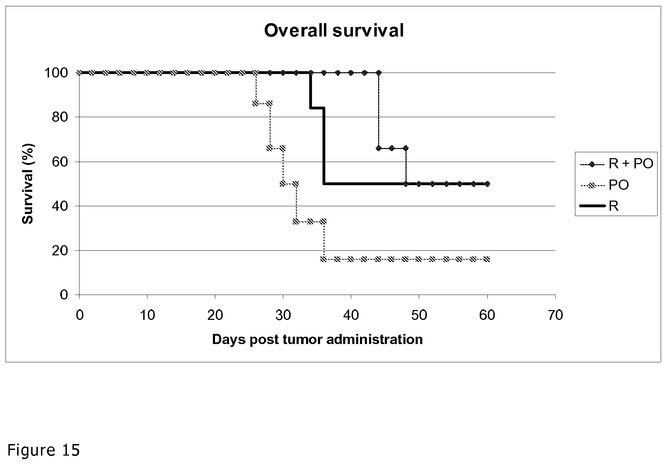

Piroctone olamine was administered by gavage with a daily dosage of 450 µg per animal in the treatment group. After the MPC11 wildtype cells were administered subcutaneously at the back of the animals on day 8, solid tumor nodules formed after a few days in 5 of 6 animals. The overall survival time was significantly (p<0.05) longer as compared to the control group (Fig. 15).

With respect to toxicity of piroctone olamine administration no side effects were observed at the applied dose.

In addition, we tested the combination of lenalidomide plus piroctone olamine in vivo. Interestingly, concerning tumor growth and survival of the animals a significant (p<0.05) additive effect was seen by the combination as compared to the single application (Fig. 15).

### Discussion

The Wnt signaling pathway has been shown to play a critical role in the early phases of B lymphocyte development. Multiple myeloma (MM) cells, but not cells from healthy donors and patients with monoclonal gammopathy of undetermined significance or other plasma cell dyscrasias involving bone marrow express the Wnt-signaling antagonist DKK1 [18]. It has been reported that secretion of DKK1 by MM cells likely contributes to the formation of osteolytic lesions in this disease by inhibiting Wnt signaling: the latter being essential for osteoblast differentiation and survival. Changes of DKK1 expression in MM cells could be traced through disturbances in the JNK signaling cascade which is differentially modulated by oxidative stress and interactions between MM cells with osteoclasts, in vitro. Despite its role as a tumor suppressor and mediator of apoptosis in other cell types, including osteoblasts, the data indicate that DKK1 - a stress-responsive gene in MM - does not mediate apoptotic signaling, is not activated by TP53, and its forced over expression could not inhibit cell growth or sensitize MM cells towards apoptosis, following treatment with either thalidomide or lenalidomide. Therefore, specific strategies may be beneficial to modulate persistent activation of the JNK pathway in preventing disease progression and treating myeloma associated bone disease by inhibiting DKK1 expression [18].

Many hypotheses have been proposed to explain the molecular mechanism of thalidomide's teratogenicity, in particular regarding limb defects. Most experimental evidence in vivo has been provided for a model suggesting the generation of oxidative stress by thalidomide with subsequent down regulation of both Wnt and Akt survival pathways. In addition, transcription factors Tbx5 and Sall4 are involved in thalidomide induced molecular pathology [19].

Since thalidomide down regulates the Wnt pathway and is used for treatment of myeloma patients, testing the combination of thalidomide and Wnt inhibitors became of particular interest. We have recently demonstrated a synergistic effect of the combination of thalidomide, PO, CIC and EA in myeloma cells. In the lymphoma cell lines SU-DHL4 and LAM-53 the Wnt inhibitors CIC and EA induce apoptosis through down regulation of beta catenin, an important molecule within the Wnt pathway.

In this study we could show that the daily oral administration of PO could lead to apoptosis of myeloma and lymphoma cells in vitro and in vivo. Combined with our previous results, our data suggest that PO, like EA and CIC may inhibit Wnt/beta catenin signaling in myeloma cell lines and thus significantly prolong the overall survival time in vivo, without deteriorating the general condition of the animals. These results might lead to new treatment options in patients with multiple myeloma and should be followed-up in further studies for the benefit of patients with myeloma.

**Table III**

| **Cell line** | **IC₅₀ thalidomide** | **IC₅₀ lenalidomide** | **IC₅₀ piroctone olamine** |
|---|---|---|---|
| OPM 2 | 53 µM | 13 µM | 0.7 µM |
| U-266 | 300 µM | 34 µM | 0.6 µM |
| RPMI 8226 | > 400 µM | 2 µM | 0.6 µM |
| KMS18 | ND | ND | 0.6 µM |
| Raji | > 400 µM | > 1000 µM | 0.6 µM |
| Oci Ly8 LAM 53 | > 400 µM | > 1000 µM | 0.6 µM |
| SU-DHL-4 | > 400 µM | > 1000 µM | 0.7 µM |
| MPC 11 | ND | ND | 0.8 µM |
| | | | |
| PBL | > 400 µM | > 200 µM | 18 µM |

**Table III:** The effect of piroctone olamine on myeloma (OPM-2, U266, RPMI 8226 and KMS18) and lymphoma (LAM53, SU-DHL-4 and Raji) cell lines was assayed. PBL derived from healthy individuals were used as controls. 1x10⁵ cells were cultured with each compound using various concentrations for three days. Then cell viability was measured by DIOC₆ staining by flow cytometry and IC 50 rates were determined. Results represent data from 2 to 4 separate experiments each. ND, not done.

### Figure legends

**Figure 12****.** Flow cytometric analysis of piroctone olamine in human OPM2 myeloma cells. 1x10⁵ cells were cultured with or without a concentration of 0.1uM piroctone olamine for three days. Then cell viability was measured by DIOC₆ staining in flow cytometry. A representative experiment is shown.
**Figure 13****.** Flow cytometric analysis of piroctone olamine in murine MPC11 myeloma cells. 1x10⁵ cells were cultured with various concentrations of piroctone olamine for three days. Then cell viability was measured by DIOC₆ staining in flow cytometry. Results represent data from two separate experiments. Data are shown as mean ± SD.
**Figure 14****.** In vivo effect of piroctone olamine (PO) in MPC11 bearing mice. PO was administered orally by gavage at a concentration of 450 µg/day/mouse. Tumor growth in mice is presented. Six animals were used in each group. The observation time was 60 days.
**Figure 15****.** In vivo effect of piroctone olamine (PO) in MPC11 bearing mice. PO was administered orally by gavage at a concentration of 450 µg/day/mouse. Overall survival of mice is presented. Six animals were used in each group. The observation time was 60 days.

### In vivo Efficacy of Griseofulvin against Multiple Myeloma

Major progress has been achieved in the treatment of multiple myeloma by the introduction of novel agents like thalidomide, lenalidomide and bortezomib. Nevertheless, multiple myeloma remains an incurable disease. In newly diagnosed patients, the combination of lenalidomide and dexamethasone has a response rate of 91%.

Several groups have shown that the Wnt/beta catenin pathway plays an important role in the regulation of cell proliferation, differentiation and apoptosis [1-3]. Aberrant activation of the Wnt signaling pathway has major oncogenic effects [4-7]. In the canonical Wnt pathway, the secreted Wnt proteins bind to a receptor complex, consisting of a member of the Frizzled (Fzd) family and the low-density lipoprotein-receptor-related proteins (LRP) 5 or LRP6. Subsequently, the cytoplasmic adaptor protein disheveled (Dvl) is phosphorylated and inhibits glycogen synthase kinase (GSK)-3β activity through its association with axin. Unphosphorylated β-catenin accumulates in the cytoplasm and translocates into the nucleus, where it interacts with T cell (TCF) and lymphoid-enhancing (LEF) factors to activate transcription of Wnt target genes [4,5,8]. In addition, it has been demonstrated that the Wnt pathway is activated in lymphoma. Thus, the Wnt/beta catenin signaling molecules are attractive candidates for development of targeted approaches in lymphoma treatment.

In a more recent work, our group confirmed that the antifungal agent ciclopirox olamine (CIC) inhibits Wnt/beta catenin signaling [9]. Ciclopirox olamine (CIC) is a synthetic antifungal agent used topically for the treatment of yeast infections in humans and is degraded by glucoronidation [10]. It serves as a chelator of polyvalent metal cations (e.g. Fe³⁺ and Al³⁺) resulting in the inhibition of metal depending enzymes, occurring in the metabolism of the cell. Furthermore, it blocks the cell cycle near the G1/S phase boundary [11].

Griseofulvin (GF), an ethanolamine salt of the hydroxamic acid derivative piroctone, is a pyridone derivate as is CIC [12]. GF, an antifungal drug derived from the mold *penicillium griseofulvum,* is used to treat fungal infections in hair and nails of both humans and animals. It binds to tubulin, interfering with microtubule function and thus inhibiting mitosis.

In this study, we investigated the effect of griseofulvin (GF) on multiple myeloma and lymphoma cells in vitro and in vivo in a murine myeloma model.

### Methods

### Cell lines and culture conditions

The lymphoma cell lines LAM-53, SU-DHL-4, Daudi and Raji, as well as the myeloma cell lines OPM-2, RPMI-8226 and U-266 (all obtained from DSMZ, Collection of Microorganisms and Cell Culture, Braunschweig, Germany) were cultured in RPMI-1640 Medium consisting of 10 % heat-inactivated fetal calf serum (FCS, Invitrogen, Karlsruhe, Germany), 2.5 % 1 M HEPES, and 1x penicillin/streptomycin (all from PAA Laboratories GmbH, Cölbe, Germany). Cells were cultured at a density of 3.3 x 10⁵ cells ml⁻¹ and incubated at 37°C with 5 % CO₂ and 95% humidity.

MPC11 (DMSZ, Braunschweig, Germany) is a murine plasmocytoma cell line derived from the Balb/c strain expressing IgG2b. Cells were cultured in RPMI 1640 medium (PAA Laboratories GmbH, Austria) supplemented with 5% fetal calf serum (FCS), 2 mM glutamine (both from PAA, Cölbe, Germany), 100 U/ml penicillin / 100 U/ml streptomycin (both from Seromed, Jülich, Germany) at 37°C in a humidified 5%CO₂ atmosphere.

### Human samples

Peripheral blood mononuclear cells (PBMC) and PBL were isolated from blood samples of healthy volunteers by Ficoll density gradient centrifugation (Lymphoprep, Nycomed, Oslo, Norway). In addition, bone marrow samples from patients with myeloma were obtained. Ethics approval had been obtained according to the guidelines of our institution.

### Drugs and chemical reagents

In our experiments the following drugs were used: Thalidomide (Grünenthal Pharma GmbH, Aachen, Germany), lenalidomide was obtained from Celgene (Munich, Germany), and griseofulvin (PO) from Sigma Aldrich, Munich, Germany. All drugs were tested at various concentrations for 24 - 72 hours. In addition, in this study griseofulvin (GF, Spinnrad, Bonn, Germany) was applied orally.

### DiOC₆ and PI-staining

1x10⁵ Cells were cultured in 3 ml Medium in 6-well plates. GF was dissolved in DMSO, and added in optimized concentrations between 10 µM (CIC) and 30 µM (EA) alone or in combination with the therapeutic agents at various concentrations for three days. The apoptosis assay was performed with 3'3-dihexyloxacarbocyanine iodide (DiOC₆) detecting mitochondrial membrane potential in viable cells, and propidium iodide (PI) which binds to DNA in necrotic cells, measured by a fluorescence-activated cell sorter (FACS).

### Fluorescence-activated cell analysis

For FACS analysis, 500µl staining solution containing 80 nM DiOC₆ in FACS buffer, consisting of deficient RPMI-medium with 0.5% bovine serum albumin (BSA), was mixed with equal volumes of the cell sample in a glass tube and incubated at 37°C for 15 min. After a washing step with PBS/BSA 1% the cells were re-suspended in 500µl PBS/BSA 1%. After addition of 5µl PI-solution (100µg/ml) the cells were analyzed by FACS. Using this assay, viable cells reveal high fluorescence intensity for DiOC₆ and a low expression for PI. In contrary, apoptotic cells show a low expression for DiOC₆ and also a low expression for PI (2). Finally, necrotic cells show a low expression for DiOC₆ and a high expression for PI.

A mean IC₅₀ value in myeloma cells was determined using the mean of the IC₅₀ results determined in OPM-2, U266 and RPMI 8226 cells.

### Isolation of peripheral blood mononuclear cells (PBMCs)

PBMCs were isolated from blood of healthy donors by Ficoll-Hypaque density gradient centrifugation. Blood from buffy coats was mixed 1:2 with PBS/1% BSA (both PAA, Cölbe, Germany) and used for a ficoll gradient (LymphoPrep, PAA, Cölbe, Germany). After the centrifugation at 800 g for 30 minutes, the leukocyte layer was removed and transferred to new tubes. Subsequently, these cells were washed 3 times with PBS/1%BSA and re-suspended in fresh medium, consisting of RPMI medium (PAA, Cölbe, Germany) with 10% fetal calf serum (Invitrogen, Karlsruhe, Germany), 2.5% hepes buffer solution (PAA, Cölbe, Germany), and 1% penicillin/streptomycin (PAA, Cölbe, Germany).

### Bone marrow samples

Bone marrow samples from patients with myeloma were taken after informed consent, single cell suspensions generated and cells ficolled. Cells were incubated with or without GF for three days and measured for viability.

### Western blot

We analyzed the effect of GF on the Wnt/beta catenin pathway by Western blot. Western blot was performed as described recently [13].

### Animal studies

All animal experiments were done at least in duplicate with groups of 6 BALB/c mice (Charles River, Sulzfeld, Germany). 5x10⁵ MCP11 murine myeloma cells were injected per mouse subcutaneously into Balb/c mice. Mice were treated orally with 450µg/day PO. Overall survival and tumor growth were measured. Tumor volume was calculated as follows: volume = length x width² x 0.52. Animals were killed when tumor volume reached 2000 mm³.

### Statistical analysis

For statistical analysis, the numbers of the results comprising the relative viability are expressed as the mean ± standard error of the mean (SEM). Different sample sizes (n) were chosen for different cell lines. Student's t test was used for statistical analysis. A p value below 0.05 was considered significant. Statistical survival analyses were performed with the software GraphPad InStat, Version 3.0.0 (GraphPad Software, San Diego, CA), applied the Mann-Whitney test (non-paired, non-parametric).

### Results

### Effect of GF in vitro on viability of various cell lines

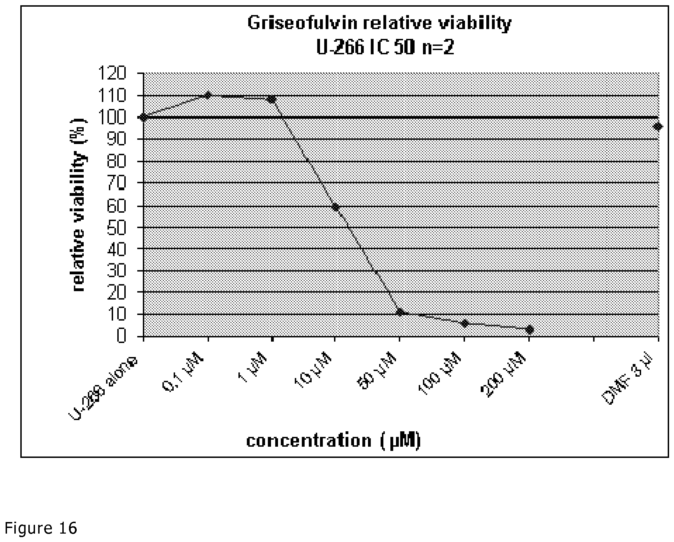

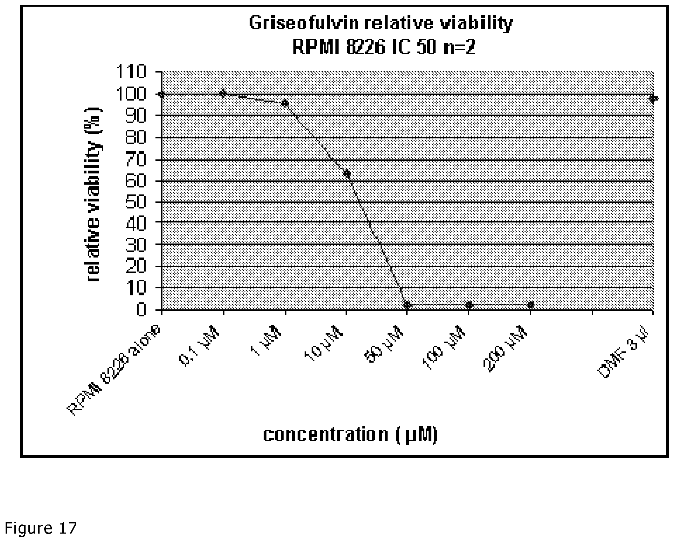

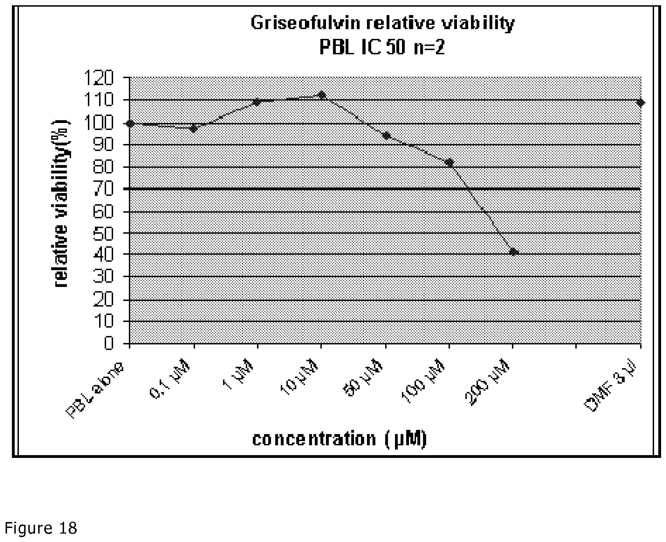

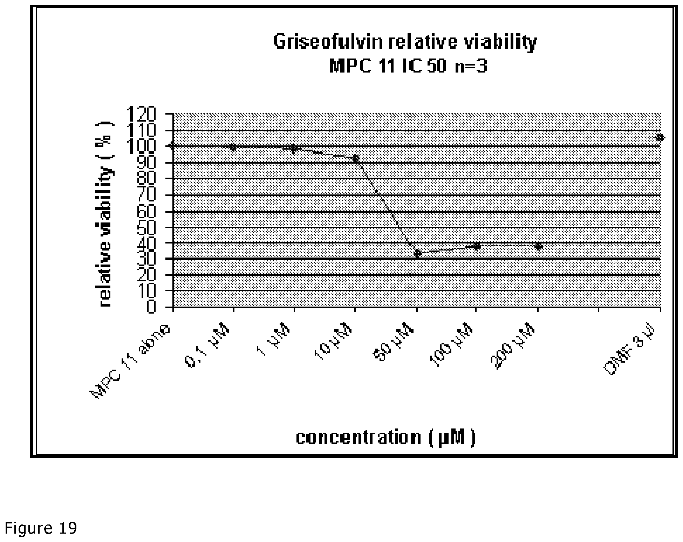

GF significantly decreased the viability of all myeloma cell lines in vitro (Table IV and Fig. 16 - 19). Even small dosages of griseofulvin were toxic, so the IC₅₀ could be reached with less than 50µM of griseofulvin for the human myeloma cell lines U-266 (18µM), RPMI-8226 (26µM) and murine MPC-11 (41µM).

Similar results were obtained for human lymphoma cell lines Raji (33µM), OCI-Ly-LAM-53 (30µM) and SU-DHL 4 (22µM) (Table IV).

In contrast, in PBMCs (180µM), which were used as control cells, griseofulvin was less efficient (Table IV).

Cytotoxic activity was shown to be via induction of apoptosis (data not shown).

### Effect of DMSO on viability of cell lines

In contrast to PBMC and lymphoma cell lines, the myeloma cell line OPM-2 was highly sensitive towards DMSO and showed a decrease of relative viability to 66.8 +/- 2.3% (data not shown).

### Effect of thalidomide, lenalidomide and GF on viability of myeloma cell lines

All three compounds significantly (p<0.05) decreased the viability of myeloma cell lines in vitro (Table IV, Fig. 16 - 19). A mean IC₅₀ value in myeloma cells was determined: Lenalidomide (16.3uM) and GF (22uM) were more effective than thalidomide (>251uM; Table IV).

In contrast the effect on normal PBL was marginal in GF and lenalidomide. Here, no effect was found for thalidomide (Table IV).

### Effect of thalidomide, lenalidomide and GF on viability of lymphoma cell lines

Only GF significantly (p<0.05) decreased the viability of lymphoma cell lines in vitro (Table IV). In contrast, lenalidomide and thalidomide showed no effect (Table IV).

### Effect of GF on the Wnt/beta catenin pathway in myeloma cells

The effect of GF on β-catenin expression in myeloma cells was tested. MPC11 cells were found to be β-catenin positive (data not shown).

### Effect of GF on viability of myeloma primary cells

Preliminary data suggest that GF inhibits the growth of primary myeloma cells derived from bone marrow from patients with multiple myeloma (data not shown).

### Effect of GF on myeloma cells in vivo

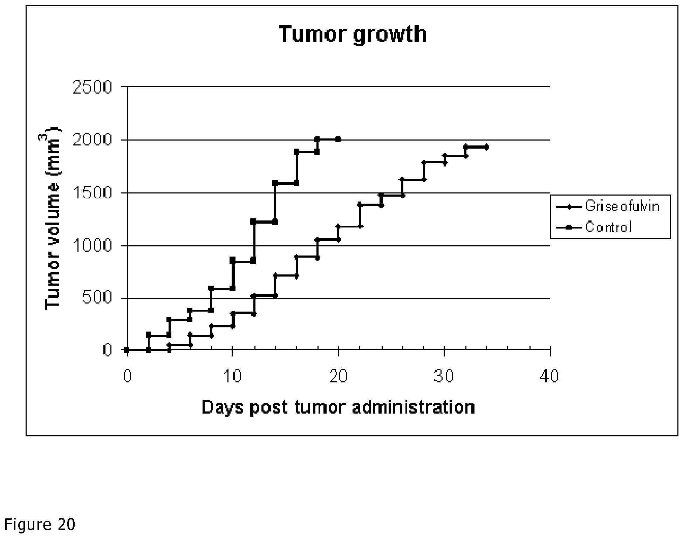

All animals in the control group developed a tumor larger than 2000 mm³ and therefore had to be sacrificed by day 22 (Fig. 20).

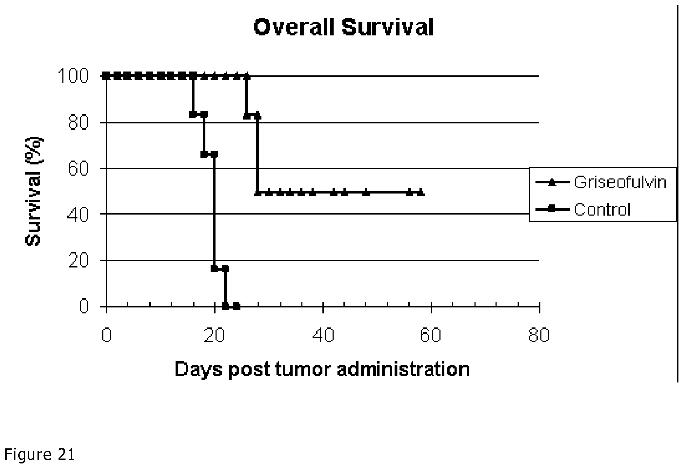

GF was administered by gavage with a daily dosage of 450 µg per animal in the treatment group. After the MPC11 wildtype cells were administered subcutaneously at the back of the animals on day 8, solid tumor nodules formed after a few days in 5 of 6 animals. The overall survival time was significantly (p<0.05) longer as compared to the control group (Fig. 21).

With respect to toxicity of GF administration no side effects were observed at the applied dose.

### Discussion

The Wnt signaling pathway has been shown to play a critical role in the early phases of B lymphocyte development. Multiple myeloma (MM) cells, but not cells from healthy donors and patients with monoclonal gammopathy of undetermined significance or other plasma cell dyscrasias involving bone marrow express the Wnt-signaling antagonist DKK1 [14]. It has been reported that secretion of DKK1 by MM cells likely contributes to the formation of osteolytic lesions in this disease by inhibiting Wnt signaling: the latter being essential for osteoblast differentiation and survival. Changes of DKK1 expression in MM cells could be traced through disturbances in the JNK signaling cascade which is differentially modulated by oxidative stress and interactions between MM cells with osteoclasts, in vitro. Despite its role as a tumor suppressor and mediator of apoptosis in other cell types, including osteoblasts, the data indicate that DKK1 - a stress-responsive gene in MM - does not mediate apoptotic signaling, is not activated by TP53, and its forced over expression could not inhibit cell growth or sensitize MM cells towards apoptosis, following treatment with either thalidomide or lenalidomide. Therefore, specific strategies may be beneficial to modulate persistent activation of the JNK pathway in preventing disease progression and treating myeloma associated bone disease by inhibiting DKK1 expression [14].

Many hypotheses have been proposed to explain the molecular mechanism of thalidomide's teratogenicity, in particular regarding limb defects. Most experimental evidence in vivo has been provided for a model suggesting the generation of oxidative stress by thalidomide with subsequent down regulation of both Wnt and Akt survival pathways. In addition, transcription factors Tbx5 and Sall4 are involved in thalidomide induced molecular pathology [15].

Since thalidomide down regulates the Wnt pathway and is used for treatment of myeloma patients, testing the combination of thalidomide and Wnt inhibitors became of particular interest. We have recently demonstrated a synergistic effect of the combination of thalidomide, PO, CIC and EA in myeloma cells. In the lymphoma cell lines SU-DHL4 and LAM-53 the Wnt inhibitors CIC and EA induce apoptosis through down regulation of beta catenin, an important molecule within the Wnt pathway.

In this study we could show that the daily oral administration of GF could lead to apoptosis of myeloma and lymphoma cells in vitro and in vivo. Combined with our previous results, our data suggest that GF, like EA and CIC may inhibit Wnt/beta catenin signaling in myeloma cell lines and thus significantly prolong the overall survival time in vivo, without deteriorating the general condition of the animals. These results might lead to new treatment options in patients with multiple myeloma and should be followed-up in further studies for the benefit of patients with myeloma.

**Table IV**

| **Cell line** | **IC₅₀ of griseofulvin** | **IC₅₀ of thalidomide** | **IC₅₀ of lenalidomide** |
|---|---|---|---|
| OPM-2 | ND | 53 µM | 13 µM |
| U266 | 18 µM | 300 µM | 34 µM |
| RPMI 8226 | 26 µM | > 400 µM | 2 µM |
| Raji | 33 µM | > 400 µM | > 1000 µM |
| OCI-Ly8 LAM 53 | 30 µM | > 400 µM | > 1000 µM |
| SU-DHL 4 | 22 µM | > 400 µM | > 1000 µM |
| MPC 11 | 41 µM | ND | ND |
| PBMC | 180 µM | > 400 µM | > 400 µM |
| PBL | 80 µM | > 400 µM | > 200 µM |

**Table IV:** The effect of griseofulvin, thalidomide,and lenalidomide on myeloma (OPM-2, U266, RPMI 8226 and KMS18) and lymphoma (LAM53, SU-DHL-4 and Raji) cell lines was assayed. PBMC and PBL derived from healthy individuals were used as controls. 1x10⁵ cells were cultured with each compound using various concentrations for three days. Then cell viability was measured by DIOC₆ staining by flow cytometry and IC 50 rates were determined. Results represent data from 2 to 4 separate experiments each.

The present invention is directed to a composition, comprising: a) one or more compounds (I) selected from the group consisting of lenalidomide, and thalidomide, and b) compound (II) ethacrynic acid, for use in the treatment of myeloma, in particular for the treatment of multiple myeloma. The present invention is further directed to the use of said compositions for the manufacture of a medicament for the treatment of myeloma, in particular for the treatment of multiple myeloma. Subject-matter which is not encompassed by the scope of the claims does not form part of the present claimed invention.

### References

1. Cadigan KM, Liu YI. Wnt signaling: complexity at the surface. J Cell Sci. 2006;119(Pt 3): 395-402.
2. Miller JR, Hocking AM, Brown JD, Moon RT. Mechanism and function of signal transduction by the Wnt/beta catenin and Wnt/Ca2+ pathways. Oncogene 1999; 18(55):7860-7872.
3. You Z, Saims D, Chen S et al. Wnt signaling promotes oncogenic transformation by inhibiting c-Myc-induced apoptosis. J Cell Biol. 2002;157(3):429-440.
4. Clevers H. Wnt/beta-catenin signaling in development and disease. Cell. 2006; 127:469-480.
5. Moon RT, Kohn AD, De Ferrari GV, Kaykas A. WNT and beta-catenin signalling: diseases and therapies. Nat Rev Genet. 2004;5:691-701.
6. Nusse R. Wnt signaling in disease and in development. Cell Res. 2005;15:28-32.
7. Polakis P. Wnt signaling and cancer. Genes Dev. 2000;14:1837-1851.
8. Willert K, Jones KA. Wnt signaling: is the party in the nucleus? Genes Dev. 2006;20: 1394-1404.
9. Lu D, Zhao Y, Tawatao R et al. Activation of the Wnt signaling pathway in chronic lymphocytic leukemia. Proc Natl Acad Sci. 2004;101:3118-3123.
10. Aizawa S, Ookawa K, Kudo T, Asano J, Hayakari M, Tsuchida S. Characterization of cell death induced by ethacrynic acid in a human colon cancer cell line DLD-1 and suppression by N-acetyl-L-cysteine. Cancer Sci. 2002;94:886-93.
11. Tew KD, Bomber AM, Hoffman SJ. Ethacrynic acid and piriprost as enhancers of cytotoxicity in drug resistant and sensitive cell lines. Cancer Res. 1988;48 (13): 3622-3625.
12. Estrela JM, Ortega A, Obrador E. Glutathione in cancer biology and therapy. Crit Rev Clin Lab Sci. 2006;43 (2):143-181.
13. McCaughan FM, Brown AL, Harrison DJ. The effect of inhibition of glutathione S-transferase P on the growth of the Jurkat human T cell line. J Pathol. 1994;172:357-362.
14. Nagourney RA, Messenger JC, Kern DH, Weisenthal LM. Enhancement of anthracycline and alkylator cytotoxicity by ethacrynic acid in primary cultures of human tissues. Cancer Chemother Pharmacol. 1990;26:318-322.
15. Hoffman BD, Hanauske-Abel HM, Flint A, Lalande M. A new class of reversible cell cycle inhibitors. Cytometry. 1991;12:26-32.
16. Lu D, Liu JX, Endo T et al. Ethacrynic acid exhibitis selective cytotoxicity to chronic Lymphocytic leukemia cells by inhibition of the Wnt/beta-catenin pathway. PloS one. 2009;14;4(12):e8294.
17. Schmidt M, Sievers E, Endo T, Lu D, Cason D, Schmidt-Wolf IGH. Targeting Wnt pathway in lymphoma and myeloma cells. Brit J Haematol. 2009;144(5):796-798.
18. Colla S, Zhan F, Xiong W et al. The oxidative stress response regulates DKK1 expression through the JNK signaling cascade in multiple myeloma plasma cells. Blood. 2007;109(10):4470-4477.
19. Knobloch J, Rüther U. Shedding light on an old mystery: thalidomide suppresses survival pathways to induce limb defects. Cell Cycle. 2008;7(9):1121-1127.
20. Fukuda Y, Nakashima S., Ujie T: The in vitro effect of a collagenocytic enzyme inhibitor on lesion development in root dentin. Am J Dent 22(2): 115 - 21, 2009.
21. Lu D, Liu JX, Endo T et al. Ethacrynic acid exhibitis selective cytotoxicity to chronic Lymphocytic leukemia cells by inhibition of the Wnt/beta-catenin pathway. PloS one, in press

## Claims

1. Composition, comprising:
a) one or more compounds (I) selected from the group consisting of:
lenalidomide, and thalidomide,
and
b) compound (II) ethacrynic acid,
for use in the treatment of myeloma.

2. Composition for use according to claim 1, wherein the composition additionally comprises ciclopirox, and/or piroctone, and/or griseofulvin.

3. Composition for use according to claims 1 or 2, wherein the composition is adapted for oral, parenteral, rectal, nasal, vaginal, and/or topical administration.

4. Composition for use according to at least one of the preceding claims, whereby compound (I) is lenalidomide and compound (II) is ethacrynic acid.

5. Composition for use according to at least one of claims 1-3, whereby compound (I) is thalidomide and compound (II) is ethacrynic acid.

6. Composition for according to at least one of the preceding claims, whereby compounds (I) are lenalidomide and thalidomide and compound (II) is ethacrynic acid.

7. Use of a composition comprising:
a) one or more compounds (I) selected from the group consisting of:
lenalidomide, and thalidomide,
and
b) compound (II) ethacrynic acid
for the manufacture of a medicament for use in the treatment of myeloma.

8. Use according to claim 7, whereby compound (I) is lenalidomide and compound (II) is ethacrynic acid.

9. Use according to claim 7, whereby compound (I) is thalidomide and compound (II) is ethacrynic acid.

10. Use according to at least one of the claims 7 to 9, whereby compounds (I) are lenalidomide and thalidomide and compound (II) is ethacrynic acid.

11. Use according to at least one of claims 7 to 10, wherein the compounds are present in dosage unit form in the medicament.

12. Use according to claims 7-8 or 10-11 wherein the dosage unit form comprises:
a) approximately 1 to 1000 mg, in particular 10 to 250 mg of lenalidomide, and
b) approximately 1 to 1000 mg, in particular 10 to 250 mg of ethacrynic acid.

13. Use according to claims 7 or 9-11, wherein the dosage unit form comprises:
a) approximately 1 to 1000 mg, in particular 10 to 250 mg of thalidomide, and
b) approximately 1 to 1000 mg, in particular 10 to 250 mg of ethacrynic acid.

14. Use according to claims 7 to 13, wherein the dosage unit form comprises:
a) approximately 1 to 1000 mg, in particular 10 to 250 mg of thalidomide, and approximately 1 to 1000 mg, in particular 10 to 250 mg of lenalidomide, and
b) approximately 1 to 1000 mg, in particular 10 to 250 mg of ethacrynic acid.

## Patentansprüche

1. Zusammensetzung, umfassend:
a) eine oder mehrere Verbindungen (I) ausgewählt aus der Gruppe, bestehend aus:
Lenalidomid und Thalidomid,
und
b) Verbindung (II) Etacrynsäure,
zur Verwendung in der Behandlung von Myelom.

2. Zusammensetzung zur Verwendung gemäß Anspruch 1, wobei die Zusammensetzung zusätzlich Ciclopirox und/ oder Pirocton und/oder Grisefulvin umfasst.

3. Zusammensetzung zur Verwendung gemäß Anspruch 1 oder 2, wobei die Zusammensetzung zur oralen, parenteralen, rektalen, nasalen, vaginale und/oder topischen Verabreichung angepasst ist.

4. Zusammensetzung zur Verwendung gemäß mindestens einem der vorhergehenden Ansprüche, wobei Verbindung (I) Lenalidomid und Verbindung (II) Etacrynsäure ist.

5. Zusammensetzung zur Verwendung gemäß mindestens einem der Ansprüche 1 bis 3, wobei Verbindung (1) Thalidomid und Verbindung (II) Etacrynsäure ist.

6. Zusammensetzung zur Verwendung gemäß mindestens einem der vorhergehenden Ansprüche, wobei die Verbindungen (I) Lenalidomid und Thalidomid sind und Verbindung (II) Etacrynsäure ist.

7. Verwendung einer Zusammensetzung umfassend:
a) eine oder mehrere Verbindungen (I) ausgewählt aus der Gruppe bestehend aus:
Lenalidomid und Thalidomid
und
b) Verbindung (II) Etacrynsäure,
zur Herstellung eines Medikaments zur Verwendung in der Behandlung von Myelom.

8. Verwendung gemäß Anspruch 7, wobei Verbindung (I) Lenalidomid und Verbindung (II) Etacrynsäure ist.

9. Verwendung gemäß Anspruch 7, wobei Verbindung (I) Thalidomid und Verbindung (II) Etacrynsäure ist.

10. Verwendung gemäß mindestens einem der Ansprüche 7 bis 9, wobei die Verbindungen (I) Lenalidomid und Thalidomid sind und Verbindung (II) Etacrynsäure ist.

11. Verwendung gemäß mindestens einem der Ansprüche 7 bis 10, wobei die Verbindungen in dem Medikament in Darreichungsform vorliegen.

12. Verwendung gemäß den Ansprüchen 7 bis 8 oder 10 bis 11, wobei die Darreichungsform
a) etwa 1 bis 1000 mg, insbesondere 10 bis 250 mg an Lenalidomid und
b) etwa 1 bis 1000 mg, insbesondere 10 bis 250 mg an Etacrynsäure,
umfasst.

13. Verwendung gemäß den Ansprüchen 7 oder 9 bis 11, wobei die Darreichungsform
a) etwa 1 bis 1000 mg, insbesondere 10 bis 250 mg an Thalidomid und
b) etwa 1 bis 1000, insbesondere 10 bis 250 mg an Etacrynsäure,
umfasst.

14. Verwendung gemäß den Ansprüchen 7 bis 13, wobei die Darreichungsform
a) etwa 1 bis 1000 mg, insbesondere 10 bis 250 mg an Thalidomid, und etwa 1 bis 1000 mg, insbesondere 10 bis 250 mg an Lenalidomid, und
b) etwa 1 bis 1000, insbesondere 10 bis 250 mg an Etacrynsäure,
umfasst.

## Revendications

1. Composition, comprenant :
a) un ou plusieurs composés (I) choisis dans le groupe constitué par :
le lénalidomide et le thalidomide,
et
b) le composé (II) acide éthacrynique,
destinée à être utilisée dans le traitement du myélome.

2. Composition destinée à être utilisée selon la revendication 1, la composition comprenant de plus du ciclopirox, et/ou de la piroctone et/ou de la griséofulvine.

3. Composition destinée à être utilisée selon les revendications 1 ou 2, la composition étant adaptée pour une administration orale, parentérale, rectale, nasale, vaginale et/ou topique.

4. Composition destinée à être utilisée selon au moins une des revendications précédentes, moyennant quoi le composé (I) est le lénalidomide et le composé (II) est l'acide éthacrynique.

5. Composition destinée à être utilisée selon au moins l'une des revendications 1 à 3, moyennant quoi le composé (I) est le thalidomide et le composé (II) est l'acide éthacrynique.

6. Composition destinée à être utilisée selon au moins une des revendications précédentes, moyennant quoi les composés (I) sont le lénalidomide et le thalidomide et le composé (II) est l'acide éthacrynique.

7. Utilisation d'une composition comprenant :
a) un ou plusieurs composés (I) choisis dans le groupe constitué par :
le lénalidomide et le thalidomide,
et
b) le composé (II) acide éthacrynique,
pour la fabrication d'un médicament destiné à être utilisé dans le traitement du myélome.

8. Utilisation selon la revendication 7, moyennant quoi le composé (I) est le lénalidomide et le composé (II) est l'acide éthacrynique.

9. Utilisation selon la revendication 7, moyennant quoi le composé (I) est le thalidomide et le composé (II) est l'acide éthacrynique.

10. Utilisation selon au moins une des revendications 7 à 9, moyennant quoi les composés (I) sont le lénalidomide et le thalidomide et le composé (II) est l'acide éthacrynique.

11. Utilisation selon au moins une des revendications 7 à 10, dans laquelle les composés sont présents sous forme de posologie unitaire dans le médicament.

12. Utilisation selon les revendications 7 à 8 ou 10 et 11, dans laquelle la forme de posologie unitaire comprend :
a) environ 1 à 1000 mg, en particulier 10 à 250 mg de lénalidomide, et
b) environ 1 à 1000 mg, en particulier 10 à 250 mg d'acide éthacrynique.

13. Utilisation selon les revendications 7 ou 9 à 11,
dans laquelle la forme de posologie unitaire comprend :
a) environ 1 à 1000 mg, en particulier 10 à 250 mg de thalidomide, et
b) environ 1 à 1000 mg, en particulier 10 à 250 mg d'acide éthacrynique.

14. Utilisation selon les revendications 7 à 13, dans laquelle la forme de posologie unitaire comprend :
a) environ 1 à 1000 mg, en particulier 10 à 250 mg thalidomide et environ 1 à 1000 mg, en particulier 10 à 250 mg de lénalidomide, et
b) environ 1 à 1000 mg, en particulier 10 à 250 mg d'acide éthacrynique.
